# DEMANDE DE BREVET EUROPEEN

(11) **EP 1 795 232 A2**
(43) Date de publication de la demande: **13.06.2007**
(21) Numéro de dépôt: 06301226.4
(22) Date de dépôt: 07.12.2006
(51) Int. Cl.: A61Q 1/04, A61K 8/37

(54) **Produit cosmétique bicouche comprenant un ester d'acide dimerdilinoléique et de polyol(s)**

(30) Priorité: 08.12.2005 FR 0553789
(71) Demandeur: L'ORÉAL, 75008 Paris (FR)
(72) Inventeur: Shimizu, Momoko, Tokyo 150-0001 (JP); Tokunaga, Emiko, Kanagawa 213-0006 (JP)
(74) Mandataire: Le Coupanec, Pascale A.M.P.

(57) **Abrégé**

La présente invention concerne un produit cosmétique de soin et/ou de maquillage de la peau et/ou des lèvres comprenant au moins :
- une première composition comprenant, dans un milieu physiologiquement acceptable, au moins un ester d'acide dimerdilinoléique et de polyol(s) ou un de ses esters, dont la viscosité, mesurée à environ 25 °C, est supérieure ou égale à environ 1 500 mPa.s, et
- une seconde composition comprenant un milieu physiologiquement acceptable.

## Description

La présente invention a pour objet des produits cosmétiques de soin et/ou de maquillage destinés à être appliqués sur la peau et/ou les lèvres comprenant au moins une première et une seconde composition, la première composition comprenant un ester d'acide dimerdilinoléique et de polyol(s) ou un de ses esters, ainsi qu'un procédé de maquillage bicouche du visage et du corps humain.

Les deux compositions du produit selon l'invention peuvent être appliquées successivement sur la peau aussi bien du visage, et notamment des lèvres, que du corps humain.

Chaque composition peut être une poudre libre ou compactée, un fond de teint, un fard à joue ou à paupières, un produit anti-cerne, un blush, un rouge à lèvres, un baume à lèvres, un brillant à lèvres, un crayon un lèvres ou à yeux, un mascara, un eye-liner, un vernis à ongles ou encore un produit de maquillage du corps ou de coloration de la peau.

De nombreuses compositions ou produits cosmétiques existent pour lesquels les propriétés de brillance et d'effet coloré du film déposé après application sur la peau et/ou les lèvres sont recherchées. Ces propriétés participent généralement à l'effet esthétique désiré. Toutefois, ces composition se heurtent généralement au problème de maintien dans le temps des effets esthétiques, et notamment du maintien de la brillance et de la couleur face aux différentes agressions auxquelles ces compositions peuvent être soumises après leur application.

Par exemple, le maintien de ces compositions, et de leurs propriétés esthétiques telles que leur effet de brillance et leur couleur, peut se trouver plus ou moins altéré après contact avec un tissu, ou après des variations de température et/ou d'humidité qui peuvent se produire au cours de la journée, ou, dans le cas des rouges à lèvres par exemple, après un repas.

De nombreuses stratégies ont été envisagées afin de conférer aux compositions et aux produits cosmétiques de bonnes propriétés de tenue, notamment de tenue de la brillance et de la couleur, au cours du temps, sans que soit affecté l'effet esthétique général qu'elles peuvent produire.

Par exemple, l'utilisation d'huiles dites « brillantes » telles que des polymères huileux comme des polybutènes de viscosité élevée, des esters d'acides ou d'alcools gras dont le nombre de carbone est élevé (typiquement supérieur à 16), ou encore de certaines huiles végétales a été envisagée afin de conférer aux compositions et produits cosmétiques des propriétés de brillance, et notamment de tenue de la brillance améliorée.

Toutefois, ces composés peuvent dans certaines circonstances présenter l'inconvénient d'être collant à l'application et dans le temps, et peuvent entraîner chez l'utilisateur de ces compositions d'importantes sensations d'inconfort.

De manière inattendue, les inventeurs ont pu observer que la mise en oeuvre de certains esters d'acides dimerdilinoléique et de polyol(s) ou d'un de ses esters, pour préparer des produits cosmétiques comprenant au moins deux compositions, l'une des deux compositions comprenant au moins un desdits esters, permettait de conférer à ces dernières une tenue de la brillance améliorée, sans affecter l'ensemble de leurs propriétés esthétiques, tout en procurant une sensation de confort à l'application.

Les inventeurs ont observé que la présence dans une première composition d'un produit cosmétique d'au moins un ester d'acide dimerdilinoléique et de polyol(s) ou d'un de ses esters permettait l'application d'une composition de nature très variable et de procurer un maquillage brillant, de tenue améliorée, tout en conservant une sensation de confort au cours du temps.

Par exemple, le maquillage obtenu peut être un maquillage bicouche.

Un maquillage bicouche peut être adapté à tous les produits de maquillage de la peau, et notamment du visage et/ou des lèvres, du corps d'être humain et des muqueuses comme les lèvres.

La seconde couche peut former des motifs et peut être appliquée avec un stylo, un crayon ou tout autre instrument (éponge, doigt, pinceau, brosse, plume, etc).

Ce maquillage peut également être appliqué sur les accessoires de maquillage, comme par exemple des pastilles ou des patches adhérant sur la peau ou les lèvres (de type mouche).

Selon un de ses aspects, la présente invention a pour objet un produit cosmétique de soin et/ou de maquillage de la peau et/ou des lèvres comprenant au moins :
- une première composition comprenant, dans un milieu physiologiquement acceptable, au moins un ester d'acide dimerdilinoléique et de polyol(s) ou un de ses esters, dont la viscosité, mesurée à environ 25 °C, est supérieure ou égale à environ 1 500 mPa.s, et
- une seconde composition comprenant un milieu physiologiquement acceptable.

Selon un de ses aspects, la présente invention a pour objet un produit cosmétique de soin et/ou de maquillage de la peau et/ou des lèvres comprenant au moins deux compositions différentes et contenant respectivement au moins un ester d'acide dimerdilinoléique et de polyol(s) ou un de ses esters, dont la viscosité mesurée à 25 °C, est supérieure ou égale à environ 2 000 mPa.s.

Selon un de ses aspects, la présente invention a pour objet un produit cosmétique de soin et/ou de maquillage de la peau et/ou des lèvres comprenant au moins deux compositions différentes avec au moins une composition contenant au moins un ester de formule générale (I) suivante :

R₃-OCO-R₁(-COO-R₂-OCO-R₁)ₙ-COO-R₃ (I)

dans laquelle :
- COR₁CO représente un résidu dimerdilinoléate,
- OR₂O représente un résidu de dimère d'alcool gras,
- OR₃ représente un résidu de monoalcool hydrocarboné, et
- n est un entier variant de 1 à 15.

Selon un de ses aspects, la présente invention a pour objet un produit cosmétique de soin et/ou de maquillage de la peau et/ou des lèvres comprenant au moins deux compositions différentes avec au moins une composition contenant au moins un ester de formule générale (II) suivante : 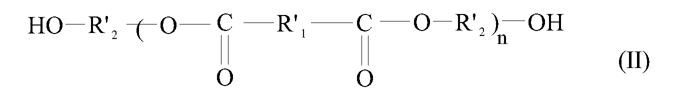 dans laquelle :
- n est un entier variant de 1 à 15,
- OCR'₁CO représente un résidu dimerdilinoléate,
- OR'₂O représente un résidu diglycéryle de formule générale (III) suivante : 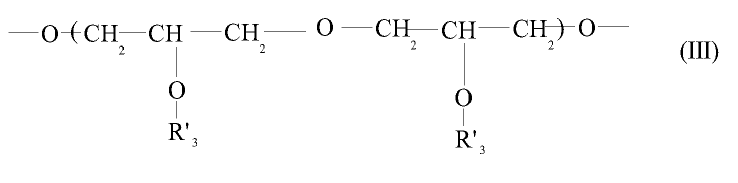
dans laquelle :
R'₃ représente H ou OR'₃ un résidu d'acide gras.
Selon un de ses aspects, la présente invention a pour objet un produit cosmétique de soin et/ou de maquillage de la peau et/ou des lèvres comprenant au moins deux compositions différentes avec au moins une composition contenant au moins un ester de formule générale (IV) :

   HO-R₁"-(-OCO-R₂''-COO-R₁''-)ₕ-OH (IV)

   dans laquelle :
   - OR₁"O représente un résidu de dimère diol obtenu par hydrogénation d'un acide dimerdilinoléique,
   - COR₂"CO représente un résidu dimerdilinoléate hydrogéné, et
   - h représente un entier variant de 1 à 9, notamment de 2 à 8, et en particulier de 4 à 6.

Selon un de ses aspects, la présente invention a pour objet un kit de maquillage comprenant au moins un produit conforme à l'invention.

Selon un de ses aspects, la présente invention a pour objet l'utilisation d'au moins un ester d'acide dimerdilinoléique et de polyol(s) ou d'un de ses esters conformes à l'invention pour la préparation d'une première composition d'un produit cosmétique de soin et/ou de maquillage de la peau et/ou des lèvres comprenant au moins deux compositions, ledit produit cosmétique présentant une tenue de la brillance améliorée.

Selon un de ses aspects, la présente invention a pour objet un procédé de maquillage et/ou de soin de la peau et/ou des lèvres comprenant au moins une étape consistant à appliquer sur au moins une partie d'un support un produit conforme à l'invention.

Selon un de ses avantages, la présente invention permet de disposer de produits cosmétiques dont la brillance, et notamment la tenue moyenne de la brillance est améliorée, tout en n'entraînant pas, chez l'utilisateur, de sensations collantes ou d'inconfort à l'application.

Selon un de ses avantages, la présente invention permet de disposer de produits cosmétiques dont la tenue de la couleur n'est pas affectée, voire est améliorée.

Au sens de la présente invention, on entend désigner par « confort » d'un produit cosmétique selon l'invention la faculté dudit produit à résister à l'écaillage et au pelage susceptibles de survenir à la suite des mouvements de la peau. Le confort d'un produit ou d'une composition cosmétique s'apprécie donc au cours du temps.

La première composition d'un produit selon l'invention peut constituer une couche de base, également nommée « base-coat » appliquée sur la peau et/ou les lèvres et la seconde composition peut constituer une couche de dessus, également nommée « top-coat », ou inversement.

Il est également possible d'appliquer sur la seconde couche une surcouche ayant la constitution ou non de la seconde couche.

Dans un souci de simplification lorsque l'expression « une ou les composition(s) conforme(s) à l'invention » est employée, elle peut désigner indifféremment la première ou la deuxième composition d'un produit conforme à l'invention.

L'objet de la présente invention peut être, par exemple, un produit cosmétique de maquillage et/ou de soin se présentant sous la forme d'un fond de teint, d'un fard à joue ou à paupières, d'un rouge à lèvres, d'un eye-liner, d'un produit anti-cerne ou d'un produit de maquillage et/ou de soin du corps.

Le produit selon l'invention peut comprendre deux (ou une pluralité de) compositions physiologiquement acceptables conditionnées séparément ou ensemble dans un même article de conditionnement ou dans deux (ou une pluralité d') articles de conditionnement séparés ou distincts.

Les compositions constituant les produits de maquillage et/ou de soin conformes à l'invention comprennent un milieu physiologiquement acceptable, notamment cosmétiquement acceptable, c'est-à-dire un milieu non toxique et compatible, par exemple, avec une application sur la peau et/ou les lèvres d'êtres humains.

### PREMIERE COMPOSITION

Une première composition peut comprendre, dans un milieu physiologiquement acceptable, au moins un ester d'acide dimerdilinoléique et de polyol(s) ou un de ses esters, dont la viscosité mesurée à environ 25 °C, est supérieure ou égale à environ 15 00 mPa.s.

L'application de cette première composition peut avoir pour objet de conférer un effet brillant et une amélioration de la tenue moyenne de la brillance, ainsi qu'un confort amélioré.

Ainsi, la surface à maquiller est préparée pour recevoir tout type de compositions cosmétiques de maquillage et/ou de soin et notamment une deuxième composition comprenant un milieu physiologiquement acceptable.

### ESTER D'ACIDE DIMERDILINOLEIQUE ET DE POLYOL(S)

Dans l'expression « ester d'acide dimerdilinoléique et de polyol(s) ou un de ses esters », on entend désigner par « ou un de ses esters », un des dérivés de ces esters acide dimerdilinoléique et de polyol(s) obtenu soit par réaction de fonction(s) alcool du polyol, non engagée(s) dans des liaisons de type ester avec des fonctions acide de l'acide dilinoléique, avec une ou plusieurs fonctions carboxylique de molécules d'acides autre que l'acide dilinoléique ou encore par réaction de fonction(s) acide du dimère dilinoléique, non engagée(s) dans des liaisons de type ester avec des fonctions alcool du polyol, avec des fonctions alcool de molécules d'alcools distinctes du polyol.

### Acide dimerdilinoléique

L'acide dimerdilinoléique convenant à la mise en oeuvre de la présente invention peut être obtenu par réaction de polymérisation, notamment de dimérisation intermoléculaire d'un acide linoléique.

La stabilité du composé vis-à-vis de l'oxydation peut être améliorée par hydrogénation des doubles liaisons restantes après la réaction de dimérisation.

L'acide dimerdilinoléique peut être également obtenu par dimérisation de la forme hydrogénée de l'acide linoléique.

La forme hydrogénée de l'acide ou du diacide peut être partielle ou totale, et par exemple correspondre à la forme saturée, plus stable à l'oxydation.

Comme indiqué précédemment, les fonctions carboxylique du résidu d'acide dimerdilinoléique non engagées dans la liaison ester avec le ou les résidus polyols peuvent être engagées dans d'autres liaisons ester avec d'autres fonctions alcool de molécules d'alcool distinctes du ou des polyol(s).

Ces molécules ou résidus d'alcools peuvent être des monoalcools ou des polyols.

A titre d'exemple de résidu d'alcool convenant à la mise en oeuvre de l'invention, on peut mentionner les composés hydrocarbonés comprenant une fonction hydroxyle et comprenant de 4 à 40 atomes de carbone, en particulier de 6 à 36 atomes de carbone, en particulier de 8 à 32 atomes de carbone, en particulier de 16 à 28 atomes de carbone, et plus particulièrement de 18 à 24 atomes de carbone.

A titre d'exemple de monoalcool convenant à l'invention, on peut citer, de manière non limitative, le butanol, le pentanol, le propanol, l'hexanol, l'heptanol, l'octanol, le décanol, le dodécanol, l'hexadécanol, l'octadécanol, l'eicosadécanol, le phytostérol, l'isostéarol, le stéarol, le cétol, le béhénol, etc.

### Polyols

Par le terme « polyol », on entend couvrir tout composé hydrocarboné comprenant au moins deux fonctions hydroxyle et comprenant de 4 à 40 atomes de carbone, en particulier de 6 à 36 atomes de carbone, en particulier de 8 à 32 atomes de carbone, en particulier de 16 à 28 atomes de carbone, et plus particulièrement de 18 à 24 atomes de carbone.

Les chaînes hydrocarbonées peuvent, le cas échéant, être interrompues par la présence d'au moins un hétéroatome, et notamment un atome d'oxygène.

Un polyol ou un ester de polyol convenant à la mise en oeuvre de la présente invention peut comprendre, par exemple, de 2 à 12 fonctions hydroxyle, en particulier de 2 à 8 fonctions hydroxyle, et plus particulièrement de 4 à 6 fonctions hydroxyle.

Le cas échéant, les fonctions hydroxyle, autres que celles déjà engagées dans une liaison ester avec l'acide dimerdilinoléique peuvent être également engagées, toutes ou en partie dans d'autres liaisons ester après réaction avec des molécules d'acides autres que l'acide dimerdilinoléique.

Le polyol ou un de ses esters convenant à la mise en oeuvre de la présente invention peut être, notamment, choisi parmi les alcools linéaires, ramifiés, cycliques ou polycycliques, saturés ou insaturés.

Ainsi, le polyol peut être choisi par exemple parmi un diol, un triol, un tétraol, ou un pentaol, ou un de leurs esters.

Le polyol peut être un diol, ou un de ses esters, par exemple choisi parmi un dimère d'alcool gras, un mono- ou poly-glycérol, un mono- ou poly-alkylène en C₂₋₄ glycol, le 1,4 butanediol, et le pentaérythritol.

A titre d'exemple de diol pouvant également convenir à la mise en oeuvre de l'invention, on peut citer, de manière non exhaustive, le butadiol, le pentadiol, le propanediol, l'hexanediol, l'hexylèneglycol, l'heptanediol, l'octanediol, le nonanediol, le décanediol, l'un-décanediol, le dodécanediol, le tridécanediol, le tétradécanediol, le pentadécanediol, l'hexadécanediol, le nonadécanediol, l'octadecènediol, le cyclohexanediol, le diglycérol, l'érythritol, le pentaérythritol, le xylitol, le sorbitol, l'éthylèneglycol, le xylèneglycol et leurs isomères.

Un dimère d'alcool gras peut également être le produit d'hydrogénation, par exemple catalytique, d'un dimère d'acide gras, lui-même obtenu par dimérisation d'un acide gras insaturé notamment en C₈ à C₃₄, notamment en C₁₂ à C₂₂, en particulier en C₁₆ à C₂₀, et plus particulièrement en C₁₈.

Un dimère d'alcool gras peut varier de C₁₆ à C₆₈, en particulier de C₂₄ à C₄₄, en particulier de C₃₂ à C₄₀ et plus particulièrement être en C₃₆.

Selon un mode de réalisation particulier, un dimère d'alcool gras peut être un dimère diol susceptible d'être le produit d'hydrogénation d'un diacide dimerdilinoléique. Il peut être sous une forme saturée.

Un dimère d'alcool gras peut, par exemple, être un dimère de dilinoléol.

A titre d'exemple de diol susceptible de convenir à la mise en oeuvre de l'invention, on peut notamment citer le diglycérol.

Ce composé est un dimère de glycérol résultant de la condensation de deux molécules de glycérol avec perte d'une molécule d'eau.

On entend par « diglycérol », l'ensemble des isomères susceptibles de résulter d'une telle condensation, comme par exemple les isomères linéaires, les isomères ramifiés et le cas échéant, les isomères cycliques résultant d'une déshydratation intra-moléculaire d'une molécule de diglycérol.

Le diglycérol peut être obtenu par tout procédé connu de l'homme de l'art et notamment ceux décrits dans le brevet EP 0 750 848.

A titre d'exemple de molécules d'acides susceptibles d'interagir avec une ou plusieurs fonctions hydroxyle du polyol, non engagée(s) dans la liaison ester avec l'acide dimerdilinoléique on peut mentionner, de manière non limitative, les molécules dérivées de l'acide isostéarique, l'acide béhénique, l'acide phytostérique, l'acide stéarique ou l'acide cétylique.

Un ester convenant à la mise en oeuvre de la présente invention peut être obtenu par réaction d'un polyol ou un de ses esters avec un acide dimerdilinoléique, selon un rapport molaire d'environ 1,0:0,2-1,0.

Un ester susceptible de convenir à la mise en oeuvre de la présente invention peut, notamment, être obtenu par réaction d'un acide dimerdilinoléique avec un dilinoléol, et le cas échéant, au moins un monoalcool additionnel, notamment choisi parmi le béhénol, l'isostéarol, le phytostérol, le stéarol, le cétol et leurs mélanges.

Ainsi, un ester mis en oeuvre dans le cadre de la présente invention peut être utilisé sous la forme d'un mélange de différents esters par exemple.

Un ester convenant à l'invention peut par exemple être obtenu par réaction d'un glycérol, d'un acide isostéarique et d'un acide dimerdilinoléique, notamment, selon un rapport molaire de 1,0:0,2-1,0:0,5-0,9.

A titre d'exemple d'ester d'acide dimerdilinoléique et de polyol(s) ou un de ses esters convenant à l'invention, on peut citer les esters décrits dans les demandes JP 2004-256515 et JP 2005-179377.

Un ester d'acide dilinoléique et de polyol(s) ou un de ses esters convenant à la mise en oeuvre de la présente invention peut présenter un poids moléculaire variant d'environ 2000 à environ 25 000 g/mol, en particulier d'environ 4 000 à environ 20 000 g/mol, en particulier d'environ 5 000 à environ 20 000 g/mol, en particulier d'environ 7 000 à environ 15 000 g/mol, et plus particulièrement d'environ 8000 à environ 10 000 g/mol.

Selon un mode de réalisation, un ester conforme à l'invention peut comprendre un enchaînement alterné de résidu(s) dimerdilinoléate(s) et de résidu(s) apparenté(s) au(x)dit(s) polyol(s), et notamment au(x)dit(s) diol(s), le(s)dit(s) polyol(s) ou diol(s) étant, par exemple, tel(s) que défini(s) précédemment.

Ainsi, dans une telle configuration, chacune des deux extrémités dudit enchaînement peut porter respectivement un motif OR' et OR", avec R' et R" représentant, indépendamment l'un de l'autre, un atome d'hydrogène ou OR' et OR" représentant, indépendamment l'un de l'autre, un résidu d'un monoalcool hydrocarboné en C₂ à C₃₆, notamment en C₈ à C₂₄, en particulier en C₁₂ à C₂₀, et plus particulièrement en C₁₆ à C₁₈.

Selon un mode de réalisation, R' et R" peuvent représenter tous les deux un atome d'hydrogène.

Selon un mode de réalisation, OR' et OR" peuvent représenter tous les deux un résidu de monoalcool hydrocarboné, identique ou différent.

A titre d'exemple de résidus de monoalcool hydrocarboné OR' et OR" pouvant convenir à l'invention, on peut mentionner les résidus d'alcools gras.

Selon un mode de réalisation, un ester d'acide dimerdilinoléique et de polyol(s) ou un de ses esters pouvant convenir à la mise en oeuvre de la présente invention peut être de formule générale (I) suivante :

R₃-OCO-R₁(-COO-R₂-OCO-R₁)ₙ-COO-R₃ (I)

dans laquelle :
- COR₁CO représente un résidu dimerdilinoléate,
- OR₂O représente un résidu de dimère d'alcool gras pouvant varier de C₁₆ à C₆₈, en particulier de C₂₄ à C₄₄, en particulier de C₃₂ à C₄₀ et plus particulièrement être en C₃₆,
- OR₃ représente un résidu de monoalcool pouvant varier de C₄ à C₄₀, en particulier de C₆ à C₃₆, en particulier de C₈ à C₃₂, en particulier de C₁₆ à C₂₈, et plus particulièrement de C₁₈ à C₂₄, et
- n est un entier variant de 1 à 15, en particulier de 2 à 10 et plus particulièrement de 5 à 7.

Selon une variante de réalisation, OR₂O peut représenter un résidu dimerdilinoléyle.

Par ailleurs, OR₃ peut représenter un résidu de monoalcool hydrocarboné choisi, par exemple, parmi les résidus béhényle, isostéaryle, phytostéryle et leurs mélanges.

Selon une autre variante de réalisation, l'ester d'acide dimerdilinoléique et de polyol(s) ou un de ses esters pouvant convenir à la mise en oeuvre de l'invention peut être, notamment, de formule générale (II) suivante : 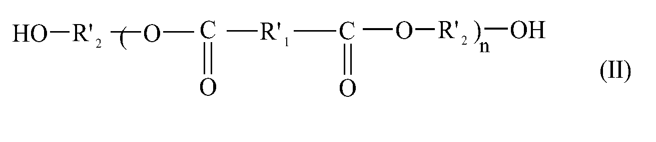 dans laquelle :
- n est un entier variant de 1 à 15, notamment de 2 à 10 et en particulier de 5 à 7,
- OCR'₁CO représente un résidu dimerdilinoléate,
- OR'₂O représente un résidu diglycéryle de formule générale (III) suivante : 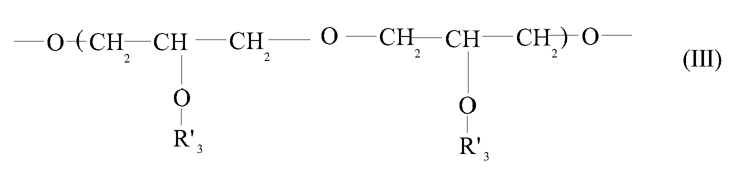
dans laquelle :
- R'₃ représente H ou OR'₃ représente un résidu d'acide gras pouvant varier de C₈ à C₃₄, en particulier de C₁₂ à C₂₂, en particulier de C₁₆ à C₃₂ et plus particulièrement étant en C₁₈.

Selon une variante de réalisation, le résidu d'acide gras figuré par OR'₃ peut être un résidu d'isostéaryle.

Selon un mode de réalisation, un ester d'acide dimerdilinoléique et de polyol(s) ou un de ses esters pouvant convenir à la mise en oeuvre de la présente invention peut être de formule (IV) suivante :

HO-R₁"-(-OCO-R₂"-COO-R₁"-)ₕ-OH (IV)

dans laquelle :
- OR₁"O représente un résidu de dimère diol obtenu par hydrogénation d'un acide dimerdilinoléique,
- COR₂"CO représente un résidu dimerdilinoléate hydrogéné, et
- h représente un entier variant de 1 à 9, et en particulier de 2 à 8, et plus particulièrement de 4 à 6.

La viscosité d'un ester d'acide dimerdilinoléique et de polyol(s) ou un de ses esters selon l'invention peut être mesurée selon tout procédé connu de l'homme de l'art, et notamment selon le procédé conventionnel décrit par la suite.

La viscosité peut être mesurée au moyen d'un viscosimètre cône/plateau ou à plaques parallèles de type ARES (TA-INSTRUMENT) et fonctionnant selon un mode en balayage de cinétique sur une gamme de cisaillement d'environ 1-1000 s⁻¹ pour induire une tension d'écoulement d'environ de 1000 Pa.

Le cône/plateau ou les plaques parallèles peuvent être consitutées d'un matériau choisi parmi l'acier inoxydable, les résines acryliques ou le sulfure de polyphénylène (résine PPS).

Le diamètre cône/plateau peut être de 25 mm (angle de cône 0,10 radiants).

La mesure est réalisée à environ 25 °C.

Avant toute mesure, la stabilité de l'échantillon est vérifiée par le test de la période de balayage dynamique qui permet de déterminer si l'échantillon est stable par lui-même.

La viscosité de cisaillement est déterminée en utilisant la valeur de ETA dans la région du plateau selon l'écoulement.

La période de balayage dynamique est déterminée à une fréquence de 1,0 Hz sur une période de 600 secondes.

Les mesures à vitesse de balayage constante sont réalisées avec une vitesse variant de 1,0 à 1000 s⁻¹, en particulier de 1,0 à 100 s⁻¹.

La viscosité d'un ester d'acide dimerdilinoléique et de polyol ou d'un de ses esters convenant à la mise en oeuvre de l'invention peut varier d'environ 1 500 mPa.s à environ 150 000 mPa.s, en particulier d'environ 2 000 mPa.s à environ 150 000 mPa.s, notamment d'environ 15 000 mPa.s à environ 100 000 mPa.s, et en particulier d'environ 30 000 mPa.s à environ 80 000 mPa.s.

Un ester convenant à l'invention peut notamment être choisi parmi les esters de nomenclature INCI suivante : le copolymère d'isostéarate de polyglyceryle-2 dimerdilinoléate, le bis-béhényl/isostéaryl/phytostéryl dimerdilinoléyle dimerdilinoléate, le dimerdilinoléyle dimerdilinoléate, et leurs mélanges.

De tels composés peuvent être obtenus, par exemple, sous la référence HAILUSCENT ISDA (KOKYU ALCOHOL), PLANDOOL-G, LUSPLAN DD-DA7, LUSPLAN PI-DA, PHY/IS-DA et LUSPLAN DD-DA5 (NIPPON FINE CHEMICAL COMPANY, Ltd).

Un ester d'acide dimerdilinoléique et de polyol(s) ou un de ses esters convenant à la mise en oeuvre de l'invention peut être présent dans les compositions cosmétiques selon l'invention en une quantité suffisante pour conférer à ces compositions des propriétés cosmétiques améliorées, notamment en terme de tenue de la brillance et de la couleur.

L'ester peut être présent en une teneur variant d'environ 5 % à environ 90 % en poids, en particulier d'environ 15 % à environ 80 % en poids et plus particulièrement d'environ 20 % à environ 50 % en poids par rapport au poids total de la composition.

### SECONDE COMPOSITION

Le produit cosmétique de maquillage et/ou de soin selon l'invention peut comprendre une seconde composition comprenant un milieu physiologiquement acceptable.

Selon un mode de réalisation, chaque composition du produit selon l'invention peut comprendre respectivement au moins un ester d'acide dimerdilinoléique et de polyol(s) ou un de ses esters, dont la viscosité mesurée à environ 25 °C, est supérieure ou égale à environ 2 000 mPa.s.

Selon un mode de réalisation, chaque composition du produit selon l'invention peut comprendre au moins un ester conforme à l'invention.

Selon un mode de réalisation, le ou les esters conformes à l'invention compris dans les compositions d'un produit selon l'invention peuvent être différents.

Selon un mode de réalisation, le milieu physiologiquement acceptable de la seconde composition peut comprendre une phase liquide non volatile à température ambiante et pression atmosphérique.

Au sens de l'invention, on entend désigner par « phase liquide non volatile » désigner tout milieu susceptible de rester sur la peau ou les lèvres pendant plusieurs heures. Une phase liquide non volatile a en particulier une pression de vapeur à température ambiante et pression atmosphérique, non nulle, inférieure à 0,02 mm de Hg (2,66 Pa), en particulier inférieure à 10⁻³ mm de Hg (0,13 Pa).

Selon un mode de réalisation, la première composition et la deuxième composition peuvent comprendre toutes les deux une phase continue de même nature.

Selon un mode de réalisation, la première ou la seconde composition, ou les deux, peuvent être à phase grasse continue, par exemple sous forme anhydre, et peuvent contenir moins de 5 % en poids d'eau, par exemple moins de 1 % en poids d'eau, par rapport au poids total de la première ou seconde composition.

Selon un mode de réalisation, le produit cosmétique de soin et/ou de maquillage bicouche entier peut être sous forme anhydre.

### MILIEU PHYSIOLOGIQUEMENT ACCEPTABLE

Les compositions d'un produit conforme à l'invention comprennent un milieu physiologiquement acceptable.

Par « milieu physiologiquement acceptable », on entend désigner un milieu convenant particulièrement à l'application d'une composition selon l'invention sur la peau et/ou les lèvres. Le milieu physiologiquement acceptable est généralement adapté à la nature du support sur lequel doit être appliquée la composition, ainsi qu'à l'aspect sous lequel la composition est destinée à être conditionnée.

Le milieu physiologiquement acceptable peut comprendre une phase aqueuse et/ou hydrosoluble et/ou une phase grasse.

Selon un mode particulier de réalisation, la phase aqueuse ou la phase grasse peut former la phase continue de la composition.

Cette phase aqueuse peut, le cas échéant, être épaissie, gélifiée ou structurée en y incorporant en outre un gélifiant aqueux traditionnel notamment d'origine minérale comme l'argile par exemple et/ou organique comme un polymère gélifiant aqueux.

Selon un autre mode particulier de réalisation, au moins une des compositions du produit conforme à l'invention peut se présenter sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de stick, de pâte, de mousse, de dispersion de vésicules notamment de lipides ioniques ou non, de lotion biphase ou multiphase ou de poudre.

Au sens de la présente invention, les émulsions contiennent une phase lipophile et une phase hydrophile, cette dernière n'étant pas systématiquement de l'eau.

Ainsi, au moins une des compositions d'un produit conforme à l'invention peut être anhydre.

En particulier, au moins une des compositions d'un produit selon l'invention peut comprendre, par exemple, une phase grasse continue, pouvant contenir moins de 10 % en poids d'eau, notamment moins de 5 % en poids d'eau, voire moins de 1 % en poids d'eau par rapport au poids total de la composition.

En particulier, au moins une des compositions d'un produit cosmétiques selon l'invention peut être anhydre, c'est-à-dire contenir moins de 5 % en poids, en particulier moins de 3 % en poids, en particulier moins de 2 % en poids, et plus particulièrement moins de 1 % en poids d'eau par rapport au poids total de la composition. Elle peut alors se présenter notamment sous forme de gels huileux, de liquides huileux, de pâtes ou de sticks ou encore sous forme de dispersion vésiculaire contenant des liquides ioniques et/ou non ioniques.

### PHASE GRASSE

Un milieu physiologiquement acceptable d'une composition cosmétique conforme à la présente invention peut comprendre au moins une phase grasse, par exemple une phase grasse liquide, choisie parmi des huiles volatiles ou non volatiles, et un corps gras solide à température ambiante (20 - 25 °C) et pression atmosphérique ; et leurs mélanges.

Un milieu physiologiquement acceptable d'une composition selon l'invention peut comprendre au moins une phase grasse liquide choisie parmi des huiles volatiles, des huiles non volatiles, et leurs mélanges.

On entend par huile, tout corps gras sous forme liquide à température ambiante (20 - 25 °C) et à pression atmosphérique. La phase grasse liquide peut, également, contenir outre des huiles, d'autres composés solubilisés dans les huiles tels que des agents gélifiants et/ou structurants.

La ou les huiles peuvent être présentes à raison de 0,1 à 99 % en poids, en particulier d'au moins 1 à 90 % en poids, plus particulièrement de 5 à 70 % en poids, notamment de 10 à 60 % en poids, voire de 20 à 50 % en poids, par rapport au poids total de la composition cosmétique selon l'invention.

La phase grasse liquide pouvant convenir à la préparation d'une composition cosmétiques selon l'invention peut être choisie parmi des huiles volatiles ou non, siliconées ou non, et leurs mélanges.

Les huiles volatiles ou non volatiles peuvent être des huiles hydrocarbonées notamment d'origine animale ou végétale, des huiles synthétiques, des huiles siliconées, des huiles fluorées, ou leurs mélanges.

Au sens de la présente invention, on entend par « huile siliconée », une huile comprenant au moins un atome de silicium, et notamment au moins un groupe Si-O.

On entend par huile hydrocarbonée, une huile contenant principalement des atomes d'hydrogène et de carbone et éventuellement des atomes d'oxygène, d'azote, de soufre et/ou de phosphore.

### Huiles volatiles

Au sens de la présente invention, on entend par « huile volatile », une huile (ou milieu non aqueux) susceptible de s'évaporer au contact de la peau en moins d'une heure, à température ambiante et à pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa (10⁻³ à 300 mm Hg), et de préférence allant de 1,3 Pa à 13 000 Pa (0,01 à 100 mm Hg), et préférentiellement allant de 1,3 Pa à 1300 Pa (0,01 à 10 mm Hg).

Les huiles hydrocarbonées volatiles peuvent être choisies parmi les huiles hydrocarbonées ayant de 8 à 16 atomes de carbone, et notamment les alcanes ramifiés en C₈-C₁₆ (appelées aussi isoparaffines) comme l'isododécane (encore appelé 2,2,4,4,6-pentaméthylheptane), l'isodécane, l'isohexadécane, et par exemple les huiles vendues sous les noms commerciaux d'ISOPARS^{®} ou de PERMETHYLS^{®}.

Comme huiles volatiles, on peut aussi utiliser les silicones volatiles, comme par exemple les huiles de silicones linéaires ou cycliques volatiles, notamment celles ayant une viscosité ≤ 8 centistokes (8 x 10⁻⁶ m²/s), et ayant notamment de 2 à 10 atomes de silicium, et en particulier de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment les diméthicones de viscosité 5 et 6 cSt, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

On peut également utiliser des huiles volatiles fluorées tels que le nonafluorométhoxybutane ou le perfluorométhylcyclopentane, et leurs mélanges.

Il est également possible d'utiliser un mélange des huiles précédemment citées.

### Huiles non volatiles

La phase grasse d'un milieu physiologiquement acceptable d'une composition cosmétique selon la présente invention peut également comprendre au moins une huile non volatile, notamment une huile de masse molaire élevée.

Au sens de la présente invention, on entend par « huile non-volatile », une huile ayant une pression de vapeur inférieure à 0,13 Pa et notamment des huiles de masse molaire élevée.

Les huiles non volatiles peuvent notamment être choisies parmi les huiles hydrocarbonées le cas échéant fluorées et/ou les huiles siliconées non volatiles.

Par « huile de masse molaire élevée », on entend des huiles ayant une masse molaire allant d'environ 650 à environ 10000 g/mol, en particulier d'environ 750 à environ 7500 g/mol, et plus particulièrement variant d'environ 1000 à environ 5000 g/mol.

Comme huile de masse molaire élevée utilisable dans la présente invention, on peut notamment citer les huiles choisies parmi :
- les polymères lipophiles,
- les esters d'acides gras linéaires ayant un nombre total de carbone allant de 35 à 70,
- les esters hydroxylés,
- les esters aromatiques,
- les esters d'alcools gras ou d'acides gras ramifiés en C₂₄-C₂₈,
- les huiles siliconées,
- les huiles d'origine végétale,
- et leurs mélanges.

Par exemple, une huile de masse molaire élevée peut être choisie parmi :
a) les polymères lipophiles tels que :
   - les polybutylènes tels que L'INDOPOL H-100 (de masse molaire MM=965 g/mol), L'INDOPOL H-300 (MM=1340 g/mol), L'INDOPOL H-1500 (MM=2160g/mol) commercialisés ou fabriqués par la société AMOCO,
   - les polyisobutylènes, par exemple, hydrogénés tels que le PANALANE H-300 E commercialisés ou fabriqué par la société AMOCO (MM =1340 g/mol), le VISEAL 20000 commercialisé ou fabriqué par la société SYNTEAL (MM=6000 g/mol), le REWOPAL PIB 1000 commercialisé ou fabriqué par la société WITCO (MM=1000 g/mol),
   - les polydécènes et les polydécènes hydrogénés tels que : le PURESYN 10 (MM=723 g/mol), le PURESYN 150 (MM=9200 g/mol) commercialisé ou fabriqués par la société MOBIL CHEMICALS,
   - les copolymères de la vinylpyrrolidone tels que : le copolymère vinylpyrrolidone/1-hexadécène, ANTARON V-216 commercialisé ou fabriqué par la société ISP (MM=7300 g/mol), et les copolymères de polyvinylpyrrolidone (PVP), tels que les copolymères d'un alkène en C₂ à C₃₀, tel qu'en C₃ à C₂₂, et des associations de ceux-ci, peuvent être utilisés. Comme exemples de copolymères de PVP pouvant être utilisés dans l'invention, on peut citer le copolymère de PVP/laurate de vinyle, de PVP/stéarate de vinyle, la polyvinylpyrrolidone butylée, de PVP/hexadécène, de PVP/triacontène ou de PVP/acide acrylique/méthacrylate de lauryle,
b) les esters tels que :
   - les esters d'acides gras linéaires ayant un nombre total de carbone allant de 35 à 70, comme le tétrapélargonate de pentaérythrityle (MM=697 g/mol),
   - les esters hydroxylés tels que le triisostéarate de polyglycérol-2 (MM=965 g/mol),
   - les esters aromatiques tels que le tridécyl trimellitate (MM=757 g/mol),
   - les esters d'alcools gras ou d'acides gras ramifiés en C₂₄-C₂₈ tels que ceux décrits dans la demande EP-A-0 955 039 et les esters du pentaérythritol, et notamment le citrate de triisoarachidyle (MM=1033,76 g/mol), le tétraisononanoate de pentaérythrityle (MM=697 g/mol), le triisostéarate de glycéryle (MM=891 g/mol), le tri décyl-2 tétradécanoate de glycéryle (MM=1143 g/mol), le tétraisostéarate de pentaérythrityle (MM=1202 g/mol), le tétraisostéarate de polyglycéryle -2 (MM=1232 g/mol) ou encore le tétra décyl -2 tétradécanoate de pentaérythrityle (MM=1538 g/mol),
   - les esters et polyesters de dimère diol, tels que les esters de dimère diol et d'acide gras, et les esters de dimère diols et de diacide, commercialisés par la société NIPPON FINE CHEMICAL et décrits dans la demande FR 03 02809 déposée le 6 mars 2003, dont le contenu est incorporé dans la présente demande par référence.
   - les huiles siliconées telles que les silicones phénylées comme la BELSIL PDM 1000 de la société WACKER (MM=9000 g/mol),
   - les huiles d'origine végétale telles que l'huile de sésame (MM=820 g/mol),
   - et leurs mélanges.

Les esters de dimère diol et d'acide mono-carboxylique peuvent être obtenus à partir d'acide mono-carboxylique comprenant de 4 à 34 atomes de carbone, notamment de 10 à 32 atomes de carbone, lesquels acides sont linéaires, ramifiés, saturés ou insaturés.

A titre illustratif des exemples d'acide mono-carboxylique convenant à l'invention, on peut notamment citer les acides gras.

Les esters de dimère diol et d'acide dicarboxylique peuvent être obtenus à partir d'un dimère diacide dérivé en particulier de la dimérisation d'un acide gras insaturé notamment en C₈ à C₃₄, notamment en C₁₂ à C₂₂, en particulier en C₁₆ à C₂₀, et plus particulièrement en C₁₈.

Selon une variante particulière, il peut s'agir plus particulièrement du dimère diacide dont dérive également le dimère diol à estérifier.

Les esters de dimère diol peuvent être obtenus à partir d'un dimère diol produit par hydrogénation catalytique d'un dimère diacide tel que décrit précédemment, par exemple le diacide dilinoléique hydrogéné.

A titre illustratif des esters de dimère diol, on peut notamment citer les esters de diacides dilinoléiques et de dimères diols dilinoléiques commercialisés par la société NIPPON FINE CHEMICAL sous la dénomination commerciale LUSPLAN DD-DA5^{®} et DD-DA7^{®}.

Une huile de masse molaire élevée peut être notamment choisie parmi les polybutylènes, les polyisobutylènes hydrogénés, les polydécènes, les polydécènes hydrogénés, les copolymères de la vinylpyrrolidone tel que le copolymère PVP/hexadécène, le tétrapélargonate de pentaérythrityle, le triisostéarate de polyglycérol-2, le tridécyl trimellitate, le citrate de triisoarachidyle, le tétraisononanoate de pentaérythrityle, le triisostéarate de glycéryle, le tri décyl-2 tétradécanoate de glycéryle, le tétraisostéarate de pentaérythrityle, le tétraisostéarate de polyglycéryle-2, le tétra décyl-2 tétradécanoate de pentaérythrityle, les silicones phénylées, l'huile de sésame, les huiles d'ester de dimer d'acide et d'alcool, et leurs mélanges.

Comme huile hydrocarbonée non volatile convenant également à la mise en oeuvre de l'invention, on peut notamment citer :
- les huiles hydrocarbonées d'origine animale,
- les huiles hydrocarbonées d'origine végétale telles que les esters de phytostéaryle, tels que l'oléate de phytostéaryle, l'isostéarate de physostéaryle et le glutanate de lauroyl/octyldodécyle/phytostéaryle par exemple vendu sous la déonmination « ELDEW PS203 » par AJINOMOTO, les triglycérides constitués d'esters d'acides gras et de glycérol dont les acides gras peuvent avoir des longueurs de chaînes variant de C₄ à C₂₄, ces dernières pouvant être linéaires ou ramifiées, saturées ou insaturées ; ces huiles sont notamment des triglycérides héptanoïques ou octanoïques, les huiles de germe de blé, de tournesol, de pépins de raisin, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de courge, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société STÉARINERIES DUBOIS ou ceux vendus sous les dénominations MIGLYOL 810^{®}, 812^{®} et 818^{®} par la société DYNAMIT NOBEL,
- les huiles d'origine minérale ou synthétique comme par exemple :
   o les éthers de synthèse ayant de 10 à 40 atomes de carbone ;
   o les hydrocarbures linéaires ou ramifiés, d'origine minérale ou synthétique tels que la vaseline, les polydécènes, le polyisobutène hydrogéné tel que le parléam, le squalane et leurs mélanges, et en particulier le polyisobutène hydrogéné,
   ○ les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10.

Les esters peuvent être notamment choisis parmi les esters, notamment d'acide gras comme par exemple :
❖ l'octanoate de cétostéaryle, les esters de l'alcool isopropylique, tels que le myristate d'isopropyle, le palmitate d'isopropyle, le palmitate d'éthyle, le palmitate de 2-éthyl-hexyle, le stéarate ou l'isostéarate d'isopropyle, l'isostéarate d'isostéaryle, le stéarate d'octyle, les esters hydroxylés comme le lactacte d'isostéaryle, l'hydroxystéarate d'octyle, l'adipate de diisopropyle, les heptanoates, et notamment l'heptanoate d'isostéaryle, octanoates, décanoates ou ricinoléates d'alcools ou de polyalcools comme le dioctanoate de propylène glycol, l'octanoate de cétyle, l'octanoate de tridécyle, le 4-diheptanoate et le palmitate d'éthyle 2-hexyle, le benzoate d'alkyle, le diheptanoate de polyéthylène glycol, le diétyl 2-d'hexanoate de propylèneglycol et leurs mélanges, les benzoates d'alcools en C₁₂ à C₁₅, le laurate d'hexyle, les esters de l'acide néopentanoïque comme le néopentanoate d'isodécyle, le néopentanoate d'isotridécyle, le néopentanoate d'isostéaryle, le néopentanoate d'octyldocécyle, les esters de l'acide isononanoïque comme l'isononanoate d'isononyle, l'isononanoate d'isotridécyle, l'isononanoate d'octyle, les esters hydroxylés comme le lactate d'isostéaryle, le malate de di-isostéaryle ;
❖ les esters de polyols, et les esters de pentaétrythritol, comme le tétrahydroxystéarate/tétraisostéarate de dipentaérythritol,
❖ les esters de dimères diols et dimères diacides tels que les Lusplan DD-DA5^{®} et Lusplan DD-DA7^{®}, commercialisés par la société NIPPON FINE CHEMICAL et décrits dans la demande FR 03 02809 déposée le 6 mars 2003,
   ○ les alcools gras liquides à température ambiante à chaîne carbonée ramifiée et/ou insaturée ayant de 12 à 26 atomes de carbone comme le 2-octyldodécanol, l'alcool isostéarylique, l'alcool oléique, le 2-hexyldécanol, le 2-butyloctanol, et le 2-undécylpentadécanol,
   ○ les acides gras supérieurs tels que l'acide oléique, l'acide linoléique, l'acide linolénique et leurs mélanges,
   ○ les carbonates de di-alkyle, les 2 chaînes alkyles pouvant être identiques ou différentes, tel que le dicaprylyl carbonate commercialisé sous la dénomination CETIOL CC^{®}, par COGNIS,
   ○ les huiles de silicone non volatiles comme par exemple les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates, les diméthicones ou phényltriméthicone de viscosité inférieure ou égale à 100 cSt, et leurs mélanges,
      - et leurs mélanges.

Les huiles non volatiles peuvent être présentes dans une composition selon l'invention en une teneur allant de 5 à 90 % en poids, notamment de 25 % à 80 % en poids, et en particulier de 40 % à 70 % en poids, par rapport au poids total de la composition.

Une huile de masse molaire élevée utilisable dans une composition selon l'invention peut être présente à raison de 5 à 40 %, de préférence de 10 à 30 %, et mieux de 15 à 20 % du poids total de la composition.

### Corps gras solides

Un milieu physiologiquement acceptable d'une composition selon l'invention peut également comprendre au moins une phase grasse solide choisie parmi les cires, les corps gras pâteux, et leurs mélanges.

La cire est solide à température ambiante (25 °C), à changement d'état solide/liquide réversible, ayant une température de fusion supérieure à 30 °C pouvant aller jusqu'à 200 °C, une dureté supérieure à 0,5 MPa et présentant à l'état solide une organisation cristalline anisotrope.

Elle peut être hydrocarbonée, fluorée et/ou siliconée et être d'origine animale, végétale, minérale ou synthétique.

Elle peut être choisie par exemple parmi la cire d'abeille, la cire de Carnauba, la cire de Candelilla, les cires de paraffine, l'huile de ricin hydrogénée, les cires synthétiques comme les cires de polyéthylène (de préférence de poids moléculaire compris entre 400 et 600) ou de Fischer-Tropsch, les cires de silicone comme les alkyl- ou alkoxy-diméthicone ayant de 16 à 45 atomes de carbone, les cérésines ou les ozokérites, comme par exemple les isoparaffines dont le point de fusion est inférieur à 40 °C, tel que l'EMW-0003, commercialisé par la société NIPPON SEIROU, les oligomères d'α-oléfine, tel que les polymères PERFORMA V^{®} 825, 103 et 260, commercialisés par la société NEW PHASE TECHNOLOGIES ; les copolymères éthylène-propylène, tel que le PERFORMALENE^{®} EP 700, et les cires microcristallines dont le point de fusion est supérieur à 85 °C, tel que les HI-MIC^{®} 1070, 1080, 1090 et 3080, commercialisées par NIPPON SEIROU, et leurs mélanges.

Selon un mode particulier de mise en oeuvre, la ou les cires utilisées dans les compositions cosmétique conformes à la présente invention est ou sont présentes en une teneur allant d'environ 5 à environ 30 %, en particulier d'environ 5 à environ 25 %, en particulier d'environ 10 à environ 20 %, et plus particulièrement d'environ 10 à environ 15 % en poids par rapport au poids total de la composition.

Un milieu physiologiquement acceptable d'une composition cosmétique conforme à la présente invention peut également comprendre au moins un composé pâteux.

Par "pâteux" au sens de la présente invention, on entend un composé gras à changement d'état solide/liquide réversible et comportant à la température de 23 °C une fraction liquide et une fraction solide. On entend également par pâteux, le polylaurate de vinyle.

Le composé pâteux au sens de l'invention présente avantageusement une dureté à 20 °C allant de 0,001 à 0,5 MPa, de préférence de 0,002 à 0,4 MPa.

Parmi les composés pâteux susceptibles d'être utilisés dans la composition selon l'invention, on peut citer les lanolines et les dérivés de lanoline comme les lanolines acétylées, les lanolines oxypropylénées ou le lanolate d'isopropyle, et leurs mélanges. On peut également utiliser des esters d'acides ou d'alcools gras, notamment ceux ayant 20 à 65 atomes de carbone comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux et leurs mélanges. Comme triglycéride d'origine végétale, on peut utiliser les dérivés d'huile de ricin hydrogénée, tels que le « THIXINR^{®} » de Rheox.

On peut également citer les polyesters résultant de l'estérification d'un acide carboxylique et d'un ester acide hydroxycarboxylique aliphatique. Par exemple, le RISOCAST^{®} DA-L (ester issu de la réaction d'estérification de l'huile de ricin hydrogéné avec de l'acide dilinoléïque dans des proportions de 2 pour 1) et le RISOCAST^{®} DA-H (ester résultant de l'estérification de l'huile de ricin hydrogénée avec de l'acide isostéarique dans des proportions de 4 pour 3) commercialisés par la société japonaise KOKYU ALCOHOL KOGYO.

Comme composé pâteux convenant avantageusement à la formulation des compositions cosmétiques conformes à la présente invention, on peut faire mention des coco-glycérides hydrogénés.

On peut aussi citer les composés pâteux siliconés tels que les polydiméthylsiloxanes (PDMS) de hauts poids moléculaires et en particulier ceux ayant des chaînes pendantes du type alkyle ou alcoxy ayant de 8 à 24 atomes de carbone, et un point de fusion de 20-55 °C, comme les stéaryl diméthicones notamment ceux vendus par la société DOW CORNING sous les noms commerciaux DC2503^{®} et DC25514^{®} et leurs mélanges.

### POLYMERE SEMI-CRISTALLIN

Un milieu physiologiquement acceptable d'une composition selon l'invention peut avantageusement comprendre en outre au moins un polymère semi-cristallin.

Par "polymère", on entend au sens de l'invention un composé comportant au moins 2 motifs de répétition, notamment au moins 3 motifs de répétition, et en particulier au moins 10 motifs répétitifs.

Par "polymère semi-cristallin", on entend au sens de l'invention, un polymère comportant une partie cristallisable et une partie amorphe dans le squelette et présentant une température de changement de phase réversible du premier ordre, en particulier sa température de fusion (transition solide-liquide). La partie cristallisable peut être soit une chaîne latérale (ou chaîne pendante), soit une séquence dans le squelette.

Lorsque la partie cristallisable du polymère semi-cristallin est une séquence du squelette polymérique, cette séquence cristallisable est de nature chimique différente de celle des séquences amorphes.

Le polymère semi-cristallin peut être dans ce cas un copolymère séquencé par exemple du type dibloc, tribloc ou multibloc.

Lorsque la partie cristallisable est une chaîne pendante au squelette, le polymère semi-cristallin peut être un homopolymère ou un copolymère.

Les séquences ou chaînes cristallisables des polymères semi-cristallins peuvent représenter au moins 30 % du poids total de chaque polymère, et mieux au moins 40%.

Par "composé organique" ou "à structure organique", on entend un composé comprenant des atomes de carbone et des atomes d'hydrogène et éventuellement des hétéroatomes comme S, O, N ou P, seuls ou en association.

Le ou les polymères semi-cristallins selon l'invention ont de préférence un point de fusion supérieur à la température du support kératinique destiné à recevoir ladite composition, en particulier la peau ou les lèvres.

Un polymère semi-cristallin convenant à l'invention peut, par exemple, posséder un point de fusion inférieur à environ 50 °C.

Le point de fusion d'un polymère semi-cristallin convenant à l'invention peut être notamment supérieur ou égal à environ 25 °C et inférieur à environ 45 °C. En particulier, le point de fusion du polymère semi-cristallin peut être supérieur ou égal à environ 35 °C et inférieur à environ 40 °C.

Les valeurs de point de fusion correspondent au point de fusion mesuré à l'aide d'un calorimètre à balayage différentiel (D.S.C), tel que le calorimètre vendu sous la dénomination DSC 30 par la société METTLER, avec une montée en température de 5 ou 10°C par minute. Le point de fusion considéré est le point correspondant à la température du pic le plus endotherme du thermogramme.

Selon l'invention les polymères semi-cristallins peuvent être solubles dans une phase grasse, notamment à au moins 1 % en poids, à une température supérieure à leur point de fusion.

Les polymères semi-cristallins de l'invention à séquences cristallisables peuvent être des copolymères, séquencés ou multiséquencés. Ils peuvent être obtenus par polymérisation de monomère à double liaisons réactives (ou éthyléniques) ou par polycondensation. Lorsque les polymères de l'invention sont des polymères à chaînes latérales cristallisables, ces derniers peuvent être avantageusement sous forme aléatoire ou statistique.

Un polymère semi-cristallin susceptible de convenir à la mise en oeuvre de la présente invention peut être choisi parmi un polymère comportant a) un squelette polymérique hydrocarboné ou siliconé et b) au moins une chaîne organique latérale cristallisable et/ou une séquence organique cristallisable faisant partie du squelette polymérique dudit polymère, ledit polymère ayant une masse moléculaire moyenne en nombre supérieure ou égale à environ 2000.

Les polymères semi-cristallins de l'invention peuvent être d'origine synthétique.

Les polymères semi-cristallins utilisables dans l'invention peuvent être choisis en particulier parmi:
- les copolymères séquencés de polyoléfines à cristallisation contrôlée, dont les monomères sont décrits dans EP-A-0 951 897.
- les polycondensats et notamment de type polyester, aliphatique ou aromatique ou aliphatique/aromatique,
- les homo- ou co-polymères portant au moins une chaîne latérale cristallisable et les homo- ou co-polymères portant dans le squelette au moins une séquence cristallisable, comme ceux décrits dans le document US-A-5 156 911,
- les homo- ou co-polymères portant au moins une chaîne latérale cristallisable en particulier à groupement(s) fluoré(s), tels que décrits dans le document WO-A-01/19333,
- les copolymère comportant des groupements carboxylates et des groupements polydiméthylsiloxanes,
- et leurs mélanges.

### A) Polymères semi-cristallins à chaînes latérales cristallisables

On peut citer en particulier ceux définis dans les documents US-A-5 156 911 et WO-A-01/19333.

Ce sont des homopolymères ou copolymères comportant de 50 à 100 % en poids de motifs résultant de la polymérisation d'un ou de plusieurs monomères porteurs de chaîne(s) latérale(s) hydrophobe(s) cristallisable(s).

Ces homo- ou co-polymères peuvent être de toute nature du moment qu'ils présentent les conditions indiquées ci-après avec, en particulier, la caractéristique d'être solubles ou dispersables dans la phase grasse, par chauffage au-dessus de leur température de fusion Pf. Ils peuvent résulter :
- de la polymérisation, notamment radicalaire, d'un ou plusieurs monomères à double(s) liaison(s) réactive(s) ou éthyléniques vis-à-vis d'une polymérisation, à savoir à groupe vinylique, (méth)acrylique ou allylique,
- de la polycondensation d'un ou plusieurs monomères porteurs de groupes co-réactifs (acide carboxylique ou sulfonique, alcool, amine ou isocyanate), comme par exemple les polyesters, les polyuréthanes, les polyéthers, les polyurées ou les polyamides.

D'une façon générale les motifs (chaînes ou séquences) cristallisables des polymères semi-cristallins selon l'invention, proviennent de monomère(s) à séquence(s) ou chaîne(s) cristallisable(s), utilisé(s) pour la fabrication des polymères semi-cristallins. Ces polymères sont choisis notamment parmi les homopolymères et copolymères résultant de la polymérisation d'au moins un monomère à chaîne(s) cristallisable(s) qui peut être représenté par la formule : 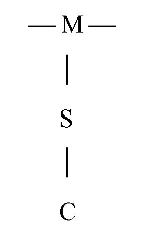
avec M représentant un atome du squelette polymérique,
S représentant un espaceur,
C représentant un groupe cristallisable.

Les chaînes « -S-C » cristallisables peuvent être aliphatiques ou aromatiques, éventuellement fluorées ou perfluorées.

« S » représente notamment un groupe (CH₂)ₙ, ou (CH₂CH₂O)ₙ ou (CH₂O), linéaire ou ramifié ou cyclique, avec n entier allant de 0 à 22.

De préférence « S » est un groupe linéaire.

De préférence, « S » et « C » sont différents.

Lorsque les chaînes cristallisables sont des chaînes aliphatiques hydrocarbonées, elles peuvent comporter des chaînes alkyle hydrocarbonées d'au moins 11 atomes de carbone et d'au plus 40 atomes de carbone et mieux d'au plus 24 atomes de carbone. Il peut s'agir notamment de chaînes aliphatiques ou de chaînes alkyle possédant au moins 12 atomes de carbone, et, en particulier, il peut s'agir de chaînes alkyle en C₁₄-C₂₄, en particulier en C₁₆-C₂₂. Lorsqu'il s'agit de chaînes alkyle fluorées ou perfluorées, elles peuvent comporter au moins 11 atomes de carbone dont au moins 6 atomes de carbone sont fluorés.

Comme exemple d'homopolymères ou de copolymères semi-cristallins à chaîne(s) cristallisable(s), on peut citer ceux résultant de la polymérisation d'un ou de plusieurs monomères choisis parmi : les (méth)acrylates d'alkyle saturés avec le groupe alkyle en C₁₄-C₂₄, les (méth)acrylates de perfluoroalkyle avec un groupe alkyle perfluoro en C₁₁-C₁₅, les N-alkyl (méth)acrylamides avec le groupe alkyle en C₁₄ à C₂₄ avec ou sans atome de fluor, les esters vinyliques à chaînes alkyle ou perfluoro (alkyle) avec le groupe alkyle en C₁₄ à C₂₄ (avec au moins 6 atomes de fluor pour une chaîne perfluoro alkyle), les éthers vinyliques à chaînes alkyle ou perfluoro (alkyle) avec le groupe alkyle en C₁₄ à C₂₄ et au moins 6 atomes de fluor pour une chaîne perfluoro alkyle, les alpha-oléfines en C₁₄ à C₂₄ comme par exemple l'octadécène, les para-alkyl styrènes avec un groupe alkyle comportant de 12 à 24 atomes de carbone, et leurs mélanges.

Lorsque les polymères résultent d'une polycondensation, les chaînes cristallisables hydrocarbonées et/ou fluorées telles que définies ci-dessus, peuvent être portées par un monomère qui peut être un diacide, un diol, une diamine ou un di-isocyanate.

Lorsque les polymères objets de l'invention sont des copolymères, ils contiennent, en plus, de 0 à 50 % de groupes Y ou Z résultant de la copolymérisation :

### α) de Y qui est un monomère polaire ou non polaire ou un mélange des deux :

Lorsque Y est un monomère polaire, il s'agit soit d'un monomère porteur de groupes polyoxyalkylénés (notamment oxyéthyléné et/ou oxypropyléné), d'un (méth)acrylate d'hydroxyalkyle comme l'acrylate d'hydroxyéthyle, le (méth)acrylamide, un N-alkyl(méth)acrylamide, un N,N-dialkyl(méth)acrylamide comme par exemple le N, N-diisopropylacrylamide ou la N-vinyl-pyrolidone (NVP), le N-vinyl caprolactame, un monomère porteur d'au moins un groupe acide carboxylique comme les acides (méth)acryliques, crotonique, itaconique, maléique, fumarique ou porteur d'un groupe anhydride d'acide carboxylique comme l'anhydre maléique, et leurs mélanges.

Lorsque Y est un monomère non polaire il peut être un ester du type (méth)acrylate d'alkyle linéaire ramifié ou cyclique, un ester vinylique, un alkyl vinyl éther, une alpha-oléfine, le styrène ou le styrène substitué par un groupe alkyle en C₁ à C₁₀, comme l'α-méthylstyrène ou un macromonomère du type polyorganosiloxane à insaturation vinylique.

Par "alkyle", on entend au sens au sens de l'invention un groupement saturé notamment en C₈ à C₂₄, sauf mention spéciale.

### β) de Z qui est un monomère polaire ou un mélange de monomères polaires.

Dans ce cas, Z a la même définition que le "Y polaire" défini ci-dessus.

De préférence, les polymères semi-cristallins à chaîne latérale cristallisable sont des homopolymères d'alkyl(méth)acrylate ou d'alkyl(méth)acrylamide avec un groupe alkyle tel que défini ci-dessus, et notamment en C₁₄-C₂₄, notamment en C₁₆-C₂₀, des copolymères de ces monomères avec un monomère hydrophile de préférence de nature différente de l'acide (méth)acrylique comme la N-vinylpyrrolidone ou l'hydroxyéthyl (méth)acrylate et leurs mélanges.

De façon avantageuse, le ou les polymères semi-cristallins à chaîne latérale cristallisable ont une masse moléculaire moyenne en poids Mp allant d'environ 5 000 à environ 1 000 000 g/mol, de préférence d'environ 10 000 à environ 800 000 g/mol, préférentiellement d'environ 15 000 à environ 500 000 g/mol, de préférence encore d'environ 100 000 à environ 200 000 g/mol.

A titre d'exemple particulier de polymère semi-cristallin utilisable dans une composition selon l'invention, on peut citer les produits Intelimer^{®} de la société Landec décrits dans la brochure "Intelimer^{®} polymers", Landec IP22 (Rev. 4-97). Ces polymères se présentent sous forme solide à température ambiante (25 °C). Ils sont porteurs de chaînes latérales cristallisables et répondent la formule générale précédente.

Par exemple, on peut choisir le produit Intelimer^{®} IPA 13-1 de la société Landec, qui est un polyacrylate de stéaryle de poids moléculaire d'environ 145 000 g/mol et dont la température de fusion est égale à environ 49 °C.

Les polymères semi-cristallins peuvent être notamment tels que ceux décrits dans les exemples 3, 4, 5, 7, 9 du brevet US-A-5 156 911 à groupement -COOH, résultant de la copolymérisation d'acide acrylique et d'alkyl(méth)acrylate en C₅ à C₁₆ de température de fusion allant de 20 °C à 35 °C et plus particulièrement de la copolymérisation :
- d'acide acrylique, d'hexadécylacrylate et d'isodécylacrylate dans un rapport 1:16:3,
- d'acide acrylique et de pentadécylacrylate dans un rapport 1:19,
- d'acide acrylique, d'hexadécylacrylate, éthylacrylate dans un rapport 2,5:76,5:20,
- d'acide acrylique, d'hexadécylacrylate et de méthylacrylate dans un rapport 5:85:10,
- d'acide acrylique, d'octadécylméthacrylate dans un rapport 2,5:97,5.

On peut aussi utiliser le polymère Structure « O » de National Starch, tel que celui décrit dans le document US-A-5 736 125, de température de fusion de 44 °C.

Les polymères semi-cristallins peuvent être notamment les polymères semi-cristallins à chaînes pendantes cristallisables comportant des groupements fluorés tels que ceux décrits dans les exemples 1, 4, 6, 7 et 8 du document WO-A-01/19333.

On peut encore utiliser les polymères semi-cristallins obtenus par copolymérisation d'acrylate de stéaryle et d'acide acrylique ou de NVP tels que décrit dans les documents US-A-5 519 063 ou EP-A- 550 745.

On peut aussi utiliser les polymères semi-cristallins obtenus par copolymérisation de l'acrylate de béhényle et de l'acide acrylique ou de NVP, tels que ceux décrits dans les documents US-A-5 519 063 et EP-A- 055 0745.

### B) Les polymères portant dans le squelette au moins une séquence cristallisable

Il peut s'agir encore de polymères solubles ou dispersables dans la phase grasse par chauffage au-dessus de leur point de fusion Pf. Ces polymères sont notamment des copolymères séquencés constitués d'au moins deux séquences de nature chimique différente dont l'une est cristallisable.

Le polymère portant dans le squelette au moins une séquence cristallisable peut être choisi parmi les copolymères séquencés d'oléfine ou de cyclooléfine à chaîne cristallisable comme ceux issus de la polymérisation séquencée de :
- cyclobutène, cyclohexène, cyclooctène, norbornène (c'est-à-dire bicyclo(2,2,1)heptène-2), 5-méthylnorbornène, 5-éthylnorbornène, 5,6-diméthylnorbornène, 5,5,6-triméthyl norbornène, 5-éthylidène-norbornène, 5-phénylnorbonène, 5-benzylnorbornène, 5-vinyl norbornène, 1,4,5,8-diméthano-1,2,3,4,4a,5,8a-octahydronaphtalène, dicyclopentadiène ou leurs mélanges, avec
- l'éthylène, le propylène, le 1-butène, le 3-méthyl-1-butène, le 1-hexène, le 4-méthyl-1-pentène, le 1-octène, le 1-décène, le 1-éicosène ou leurs mélanges,
- et en particulier les copoly(éthylène/norbornène) blocs et les terpolymères (éthylène/propylène/éthylidène-norbornène) blocs.

On peut aussi utiliser ceux résultants de la copolymérisation séquencée d'au moins 2 α-oléfines en C₂-C₁₆ et mieux en C₄-C₁₂ tels que ceux cités précédemment et en particulier les bipolymères séquencés d'éthylène et d'1-octène.

Le polymère portant dans son squelette au moins une séquence cristallisable peut être choisi parmi les copolymères présentant au moins une séquence cristallisable, le reste du copolymère étant amorphe à température ambiante. Ces copolymères peuvent, en outre, présenter deux séquences cristallisables de nature chimique différente.

Les copolymères préférés sont ceux qui possèdent à la fois, à température ambiante, une séquence cristallisable et une séquence amorphe à la fois hydrophobe et lipophile réparties séquentiellement. On peut citer par exemple les polymères possédant une des séquences cristallisables et une des séquences amorphes suivantes :
- séquence cristallisable par nature de type polyester comme les poly(alkylène téréphtalate), ou de type polyoléfine comme les polyéthylènes ou polypropylènes,
- séquence amorphe et lipophile comme les polyoléfines ou copoly(oléfine)s amorphes telles que le poly(isobutylène), le polybutadiène hydrogéné ou le poly(isoprène) hydrogéné.

Comme exemple de tels copolymères à séquence cristallisable et à séquence amorphe, on peut citer :
α) les copolymères séquencés poly(ε-caprolactone)-*b*-poly(butadiène), utilisés de préférence hydrogénés, tels que ceux décrits dans l'article "Melting behavior of poly(-caprolactone)-block-polybutadiène copolymers" de S. Nojima, Macromolécules, 32, 3727-3734 (1999).
β) les copolymères séquencés poly(butylènetéréphtalate)-*b*-poly(isoprène) hydrogénés séquencés ou multiséquencés, cités dans l'article "Study of morphological and mechanical properties of PP/PBT" de B. Boutevin et al., Polymer Bulletin, 34, 117-123 (1995).
γ) les copolymères séquencés poly(éthylène)-*b*-copoly(éthylène/propylène) cités dans les articles "Morphology of semi-crystalline block copolymers of ethylene-(ethylene-alt-propylene)" de P. Rangarajan et al., Macromolecules, 26, 4640-4645 (1993) et "Polymer agregates with crystalline cores : the system poly(ethylene)-poly(ethylene-propylene)" P. Richter et al., Macromolécules, 30, 1053-1068 (1997).
δ) les copolymères séquencés poly(éthylène)-*b*-poly(éthyléthylène) cités dans l'article général "Cristallization in block copolymers" de I.W. Hamley, Advances in Polymer Science, vol 148, 113-137 (1999).

### C) Polycondensats de type polyester, aliphatique ou aromatique ou aliphatique/aromatique

Les polycondensats polyester peuvent être choisis parmi les polyesters aliphatiques. Leur masse moléculaire est par exemple supérieure ou égale à environ 200 g/mol et inférieure ou égale à environ 15000 g/mol, et en particulier supérieure ou égale à environ 1000 g/mol et inférieure ou égale à environ 10000 g/mol, en particulier supérieure ou égale à environ 2000 g/mol et inférieure ou égale à environ 5000 g/mol.

Les polycondensats polyester sont en particulier choisis parmi les polycaprolactones. En particulier, les polycaprolactones peuvent être choisies parmi les homopolymères d'ε-caprolactones. L'homopolymérisation peut être initiée avec un diol, notamment un diol ayant de 2 à 10 atomes de carbone, tels que le diéthylène glycol, le 1,4-butanediol ou le néopentyl glycol.

On peut utiliser par exemple les polycaprolactones, notamment celles commercialisées sous la dénomination de CAPA^{®} 240 (point de fusion de 68 °C et poids moléculaire de 4000 g/mol), 223 (point de fusion de 48 °C et poids moléculaire de 2000 g/mol), 222 (point de fusion de 48 °C et poids moléculaire de 2000 g/mol), 217 (point de fusion de 44 °C et poids moléculaire de 1250 g/mol), 2125 (point de fusion de 45 °C et poids moléculaire de 1250 g/mol), 212 (point de fusion de 45 °C et poids moléculaire de 1000 g/mol), 210 (point de fusion de 38 °C et poids moléculaire de 1000 g/mol), 205 (point de fusion de 39 °C et poids moléculaire de 830 g/mol) par la société SOLVAY, ou PCL-300, PCL-700 par la société UNION CARBIDE.

On peut utiliser en particulier la CAPA^{®} 2125 dont la température de fusion est comprise entre 35 et 45 °C et dont la masse moléculaire en poids est égale à 1250 g/mol.

### D) Copolymère comportant des groupements carboxylates et des groupements polydiméthylsiloxanes

Par « copolymère comportant des groupements carboxylates et des groupements polydiméthylsiloxanes », on entend dans la présente demande, un copolymère résultant de la polymérisation (a) d'un ou plusieurs monomère(s) carboxylique(s) (acide ou ester), avec (b) une ou plusieurs chaîne(s) polydiméthylsiloxane (PDMS) comportant au moins un radical polymérisable.

Parmi les « monomères carboxyliques » convenant à la mise en oeuvre de l'invention, on peut citer les monomères d'acide carboxylique et les monomères d'ester d'acide carboxylique.

Ainsi, le monomère (a) peut être choisi par exemple parmi l'acide acrylique, l'acide méthacrylique, l'acide maléique, l'acide fumarique, l'acide itaconique, l'acide crotonique, leurs esters et les mélanges de ces monomères. Comme esters, on peut citer les monomères suivants : les acrylates, méthacrylates, maléates, fumarates, itaconoates et/ou crotonoates de métyle, d'éthyle, de stéaryle, de butyle, d'éthyl-2-héxyle et leurs mélanges. Selon un mode de réalisation de l'invention, les monomères sous forme d'esters peuvent être choisis parmi les acrylates et/ou méthacrylates d'alkyle linéaire ou ramifié, de préférence en C₁-C₂₄ et mieux en C₁-C₂₂, le radical alkyle étant choisi parmi les radicaux méthyle, éthyle, stéaryle, butyle, éthyl-2-hexyle, et leurs mélanges.

Selon un mode de réalisation de l'invention, le copolymère peut comprendre comme groupements carboxylates, au moins un groupement choisi parmi l'acide acrylique, l'acide méthacrylique, les acrylates ou méthacrylates de méthyle, d'éthyle, de stéaryle, de butyle, d'éthyl-2-hexyle, et leurs mélanges.

Dans la présente demande, on entend désigner par « polydiméthylsiloxanes » (appelé aussi organopolysiloxanes ou, en abréviation, PDMS), en conformité avec l'acceptation générale, tout polymère ou oligomère organosilicié à structure linéaire, de poids moléculaire variable, obtenus par polymérisation et/ou polycondensation de silanes convenablement fonctionnalisés, et constitués, pour l'essentiel, par une répétition de motifs principaux dans lesquels les atomes de silicium sont reliés entre eux par des atomes d'oxygène (liaison siloxane ≡Si-O-Si≡), comportant des radicaux triméthyle directement liés par l'intermédiaire d'un atome de carbone sur lesdits atomes de silicium.

Les chaînes PDMS pouvant être utilisées pour obtenir le copolymère utilisé selon l'invention comportent au moins un groupe radical polymérisable, de préférence situé sur au moins l'une des extrémités de la chaîne, c'est-à-dire que le PDMS peut avoir par exemple un groupe radical polymérisable sur les deux extrémités de la chaîne ou avoir un groupe radical polymérisable sur une extrémité de la chaîne et un groupement terminal triméthylsilyle sur l'autre extrémité de la chaîne. Le groupe radical polymérisable peut être notamment un groupe acrylique ou méthacrylique, en particulier un groupe CH₂ = CR₁-CO-O-R₂, où R₁ représente un hydrogène ou un groupe méthyle, et R₂ représente -CH₂-, -(CH₂)n- avec n = 3, 5, 8 ou 10, -CH₂-CH(CH₃)-CH₂- , -CH₂-CH₂-O-CH₂-CH₂-, - CH₂-CH₂₋O-CH₂-CH₂-CH(CH₃)-CH₂-, -CH₂-CH₂-O-CH₂-CH₂-O-CH₂-CH₂-CH₂-.

Les copolymères utilisables dans la composition de l'invention peuvent être obtenus selon les méthodes usuelles de polymérisation et de greffage, par exemple par polymérisation radicalaire (a) d'un PDMS comportant au moins un groupe radical polymérisable (par exemple sur l'une des extrémités de la chaîne ou sur les deux) et (b) d'au moins un monomère carboxylique, comme décrit par exemple dans les documents US-A-5,061,481 et US-A-5,219,560.

Les copolymères obtenus peuvent avoir un poids moléculaire allant d'environ 3000 g/mol à environ 200 000 g/mol et notamment d'environ 5000 g/mol à environ 100 000 g/mol.

Un copolymère utilisable dans une composition de l'invention peut se présenter tel quel, ou sous forme dispersée dans un solvant, tel que les alcools inférieurs comportant de 2 à 8 atomes de carbone, comme l'alcool isopropylique, ou les huiles comme les huiles de silicone volatiles (par exemple cyclopentasiloxane).

Comme copolymères utilisables dans une composition de l'invention, on peut citer par exemple les copolymères d'acide acrylique et d'acrylate de stéaryle à greffons polydiméthylsiloxane, les copolymères de méthacrylate de stéaryle à greffons polydiméthylsiloxane, les copolymères d'acide acrylique et de méthacrylate de stéaryle à greffons polydiméthylsiloxane, les copolymères de méthacrylate de méthyle, méthacrylate de butyle, d'acrylate d'éthyl-2-hexyle et de méthacrylate de stéaryle à greffons polydiméthylsiloxane. On peut également citer comme copolymère utilisable dans la composition de l'invention, les copolymères commercialisés par la société SHIN-ETSU sous les dénominations KP-561 (nom CTFA: acrylates/dimethicone), KP-541 où le copolymère est dispersé à 60 % en poids dans de l'alcool isopropylique (nom CTFA : acrylates/dimethicone and Isopropyl alcohol), KP-545 où le copolymère est dispersé à 30 % dans du cyclopentasiloxane (nom CTFA : acrylates/dimethicone and Cyclopentasiloxane).

Selon un mode particulier de réalisation de l'invention, on peut utiliser un copolymère d'acrylate et de diméthicone, tel que le KP-561 ; ce copolymère n'est pas dispersé dans un solvant, mais se présente sous forme cireuse, son point de fusion étant d'environ 30 °C.

Dans les deux derniers cas, la ou les chaînes latérales ou séquences cristallisables sont hydrophobes.

Les polymères semi-cristallins de la composition de l'invention peuvent être réticulés ou non à partir du moment où le taux de réticulation ne gène pas leur dissolution ou dispersion dans la phase grasse par chauffage au-dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère comme par exemple les interactions dipolaires entre ionomères carboxylates, ces interactions étant en faible quantité et portées par le squelette du polymère ; soit à une séparation de phase entre les séquences cristallisables et les séquences amorphes, portées par le polymère.

De préférence, les polymères semi-cristallins de la composition selon l'invention sont non réticulés.

En pratique, la quantité totale de polymère(s) semi-cristallin(s) peut représenter d'environ 1 à environ 8 % en poids du poids total de la composition, en particulier d'environ 2 à environ 6 % en poids et plus particulièrement d'environ 3 à environ 6 % en poids. En particulier, il peut représenter d'environ 4 à environ 5 % en poids du poids total de la composition.

Selon un mode de réalisation, l'ester d'acide dimerdilinoléique et de polyol(s) ou un de ses esters, et le polymère semi-cristallin peuvent être présents, dans les compositions selon l'invention, selon un rapport pondéral pouvant varier d'environ 15:1 à environ 2:1, en particulier d'environ 10:1 à environ 4:1 et plus particulièrement d'environ 8:1 à environ 6:1.

### MATIERE COLORANTE

Un milieu physiologiquement acceptable d'une composition cosmétique conforme à l'invention peut, en outre, incorporer au moins une matière colorante.

Une telle matière colorante peut être choisie parmi une matière colorante hydrosoluble, ou non, liposoluble, ou non, organique ou inorganique, par exemple de type pigments ou nacres, classiquement utilisée dans les compositions cosmétiques.

Par pigments, il faut comprendre des particules blanches ou colorées, minérales ou organiques, insolubles dans une solution aqueuse, destinées à colorer et/ou opacifier le film résultant.

Les pigments peuvent être présents à raison de 0,5 à 30 % en poids, notamment de 5 à 25 % en poids, et en particulier de 10 à 20 % en poids, par rapport au poids total de la composition cosmétique.

Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome.

Il peut également s'agir de pigment ayant une structure qui peut être par exemple de type séricite/oxyde de fer brun/dioxyde de titane/silice. Un tel pigment est commercialisé par exemple sous la référence COVERLEAF NS ou JS par la société CHEMICALS AND CATALYSTS et présente un rapport de contraste voisin de 30.

La matière colorante peut encore comporter un pigment ayant une structure qui peut être par exemple de type microsphères de silice contenant de l'oxyde de fer. Un exemple de pigment présentant cette structure est celui commercialisé par la société MIYOSHI sous la référence PC BALL PC-LL-100 P, ce pigment étant constitué de microsphères de silice contenant de l'oxyde de fer jaune.

Parmi les pigments organiques utilisables dans l'invention, on peut citer le noir de carbone, les pigments de type D & C, les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537.

Par « nacres », il faut comprendre des particules colorées de toute forme, irisées ou non, notamment produites par certains mollusques dans leur coquille ou bien synthétisées et qui présentent un effet de couleur par interférence optique.

Les nacres peuvent être choisies parmi les pigments nacrés tels que le mica titane recouvert avec un oxyde de fer, le mica titane recouvert d'oxychlorure de bismuth, le mica titane recouvert avec de l'oxyde de chrome, le mica titane recouvert avec un colorant organique ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

On peut également citer, à titre d'exemple de nacres, le mica naturel recouvert d'oxyde de titane, d'oxyde de fer, de pigment naturel ou d'oxychlorure de bismuth.

Parmi les nacres disponibles sur le marché, on peut citer les nacres TIMICA, FLAMENCO et DUOCHROME (sur base de mica) commercialisées par la société ENGELHARD, les nacres TIMIRON commercialisées par la société MERCK, les nacres sur base de mica PRESTIGE commercialisées par la société ECKART et les nacres sur base de mica synthétique SUNSHINE commercialisées par la société SUN CHEMICAL.

Les nacres peuvent plus particulièrement posséder une couleur ou un reflet jaune, rose, rouge, bronze, orangé, brun, or et/ou cuivré.

A titre illustratif des nacres pouvant être mises en oeuvre dans le cadre de la présente invention, on peut notamment citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

La composition cosmétique selon l'invention peut comprendre également au moins une matière colorante hydrosoluble ou liposoluble en une teneur allant de 0,5 à 30 % en poids, notamment allant de 5 à 25 % en poids par rapport au poids total de la composition cosmétique.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine Les colorants hydrosolubles sont par exemple le jus de betterave, le bleu de méthylène.

La composition cosmétique selon l'invention peut également contenir au moins un matériau à effet optique spécifique.

Cet effet est différent d'un simple effet de teinte conventionnel, c'est-à-dire unifié et stabilisé tel que produit par les matières colorantes classiques comme par exemple les pigments monochromatiques.

Au sens de l'invention, « stabilisé » signifie dénué d'effet de variabilité de la couleur avec l'angle d'observation ou encore en réponse à un changement de température.

Par exemple, ce matériau peut être choisi parmi les particules à reflet métallique, les agents de coloration goniochromatiques, les pigments diffractants, les agents thermochromes, les agents azurants optiques, ainsi que les fibres, notamment interférentielles. Bien entendu, ces différents matériaux peuvent être associés de manière à procurer la manifestation simultanée de deux effets, voire d'un nouvel effet conforme à l'invention.

Les particules à reflet métallique utilisables dans l'invention sont en particulier choisies parmi :
- les particules d'au moins un métal et/ou d'au moins un dérivé métallique,
- les particules comportant un substrat, organique ou minéral, monomatière ou multimatériaux, recouvert au moins partiellement par au moins une couche à reflet métallique comprenant au moins un métal et/ou au moins un dérivé métallique, et
- les mélanges desdites particules.

Parmi les métaux pouvant être présents dans lesdites particules, on peut citer par exemple Ag, Au, Cu, Al, Ni, Sn, Mg, Cr, Mo, Ti, Zr, Pt, Va, Rb, W, Zn, Ge, Te, Se et leurs mélanges ou alliages. Ag, Au, Cu, Al, Zn, Ni, Mo, Cr, et leurs mélanges ou alliages (par exemple les bronzes et les laitons) sont des métaux préférés.

Par « dérivés métalliques », on désigne des composés dérivés de métaux notamment des oxydes, des fluorures, des chlorures et des sulfures

A titre illustratif de ces particules, on peut citer des particules d'aluminium, telles que celles commercialisées sous les dénominations STARBRITE 1200 EAC^{®} par la société SIBERLINE et METALURE^{®} par la société ECKART.

On peut également citer les poudres métalliques de cuivre ou des mélanges d'alliage telles les références 2844 commercialisées par la société RADIUM BRONZE, les pigments métalliques comme l'aluminium ou le bronze, telles que celles commercialisées sous les dénominations ROTOSAFE 700 de la société ECKART, les particules d'aluminium enrobé de silice commercialisées sous la dénomination VISIONAIRE BRIGHT SILVER de la société ECKART et les particules d'alliage métallique comme des poudres de bronze (alliage cuivre et zinc) enrobé de silice commercialisées sous la dénomination de Visionaire Bright Natural Gold de la société Eckart.

Il peut encore s'agir de particules comportant un substrat de verre comme celles commercialisées par la société NIPPON SHEET GLASS sous les dénominations MICROGLASS METASHINE.

L'agent de coloration goniochromatique peut être choisi par exemple parmi les structures multicouches interférentielles et les agents de coloration à cristaux liquides.

Des exemples de structures multicouche interférentielles symétriques utilisables dans des compositions réalisées conformément à l'invention sont par exemple les structures suivantes : Al/SiO₂/Al/SiO₂/Al, des pigments ayant cette structure étant commercialisés par la société DUPONT DE NEMOURS ; Cr/MgF₂/Al/MgF₂/Cr, des pigments ayant cette structure étant commercialisés sous la dénomination CHROMAFLAIR par la société FLEX ; MoS₂/SiO₂/Al/SiO₂/MoS₂ ; Fe₂O₃/SiO₂/Al/SiO₂/Fe₂O₃, et Fe₂O₃/SiO₂/Fe₂O₃/SiO₂/Fe₂O₃, des pigments ayant ces structures étant commercialisés sous la dénomination SICOPEARL par la société BASF ; MoS₂/SiO₂/mica-oxyde/SiO₂/MoS₂ ; Fe₂O₃/SiO₂/mica-oxyde/SiO₂/Fe₂O₃ ; TiO₂/SiO₂/TiO₂ et TiO₂/Al₂O₃/TiO₂ ; SnO/TiO₂/SiO₂/TiO₂/SnO ; Fe₂O₃/SiO₂/Fe₂O₃ ; SnO/mica/TiO₂/SiO₂/TiO₂/mica/SnO, des pigments ayant ces structures étant commercialisés sous la dénomination XIRONA par la société MERCK (Darmstadt). A titre d'exemple, ces pigments peuvent être les pigments de structure silice/oxyde de titane/oxyde d'étain commercialisés sous le nom XIRONA MAGIC par la société MERCK, les pigments de structure silice/oxyde de fer brun commercialisés sous le nom XIRONA INDIAN SUMMER par la société MERCK et les pigments de structure silice/oxyde de titane/mica/oxyde d'étain commercialisés sous le nom XIRONA CARRIBEAN BLUE par la société MERCK. On peut encore citer les pigments INFINITE COLORS de la société SHISEIDO. Selon l'épaisseur et la nature des différentes couches, on obtient différents effets. Ainsi, avec la structure Fe₂O₃/SiO₂/Al/ SiO₂/Fe₂O₃ on passe du doré-vert au gris-rouge pour des couches de SiO₂ de 320 à 350 nm ; du rouge au doré pour des couches de SiO₂ de 380 à 400 nm ; du violet au vert pour des couches de SiO₂ de 410 à 420 nm ; du cuivre au rouge pour des couches de SiO₂ de 430 à 440 nm.

On peut citer, à titre d'exemple de pigments à structure multicouche polymérique, ceux commercialisés par la société 3M sous la dénomination COLOR GLITTER.

Comme particules goniochromatiques à cristaux liquides, on peut utiliser par exemple celles vendues par la société CHENIX ainsi que celle commercialisées sous la dénomination HELICONE^{®} HC par la société WACKER.

Selon un mode de réalisation, une matière colorante convenant à l'invention peut être choisie parmi les matières colorantes organiques, les matières colorantes inorganiques, telles que les pigments et les nacres, les matériaux à effet optique spécifique, et leurs mélanges.

Selon un mode de réalisation, une composition selon l'invention peut ne pas comprendre plus de 30 % en poids de matière colorante par rapport au poids total de la composition.

### CHARGES

Les compositions cosmétiques conformes à l'invention peuvent également comprendre au moins une charge, de nature organique ou minérale, permettant notamment de leur conférer une stabilité améliorée au regard de l'exsudation.

Par « charge », il faut comprendre les particules incolores ou blanches, solides de toutes formes, qui se présentent sous une forme insoluble et dispersée dans le milieu de la composition. De nature minérale ou organique, elles permettent de conférer du corps ou de la rigidité à la composition, et/ou de la douceur, de la matité et de l'uniformité au maquillage.

Les charges utilisées dans les compositions selon la présente invention peuvent être de formes lamellaires, globulaires, sphériques, de fibres ou de toute autre forme intermédiaire entre ces formes définies.

Les charges selon l'invention peuvent être ou non enrobées superficiellement, et en particulier elles peuvent être traitées en surface par des silicones, des acides aminés, des dérivés fluorés ou toute autre substance favorisant la dispersion et la compatibilité de la charge dans la composition.

Au sens de la présente invention, les termes « charges minérales » et « charges inorganiques » sont utilisés de manière interchangeable.

Parmi les charges minérales utilisables dans les compositions selon l'invention, on peut citer le talc, le mica, la silice, le siloxysilicate de triméthyle, le kaolin, la bentone, le carbonate de calcium précipité, le carbonate et l'hydrogéno-carbonate de magnésium, l'hydroxyapatite, le nitrure de bore, les microsphères de silice creuses (Silice Beads de Maprecos), les microcapsules de verre ou de céramique, les charges à base de silice comme l'Aerosil 200, l'Aerosil 300 ; le Sunsphare L-31, le Sunphare H-31 commercialisés par Asahi Glass ; le Chemicelen commercialisé par Asahi Chemical ; les composites de silice et de dioxyde de titane comme la série TSG commercialisée par Nippon Sheet Glass, et leurs mélanges.

Parmi les charges organiques utilisables dans les compositions selon l'invention on peut citer les poudres de polyamide (Nylon^{®} Orgasol de chez Atochem), de poly-b-alanine et polyéthylène, les poudres de polytétrafluoroéthylène (Téflon®), la lauroyl-lysine, l'amidon, les poudres de polymères de tétrafluoroéthylène, les microsphères creuses de polymères telles l'EXPANCEL (NOBEL INDUSTRIE), le carbonate de calcium précipité, le carbonate et l'hydrogéno-carbonate de magnésium, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, le Polypore® L 200 (Chemdal Corporation), les microbilles de résine de silicone (Tospearl ® de Toshiba, par exemple), les poudres de poyuréthanne, en particulier les poudres de polyuréthanne réticulé comprenant un copolymère, ledit copolymère comprenant du triméthylol hexyllactone. En particulier, il peut s'agir d'un polymère d'hexaméthylène di-isocyanate/triméthylol hexyllactone. De telles particules sont notamment disponibles dans le commerce, par exemple sous la dénomination de PLASTIC POWDER D-400^{®} ou PLASTIC POWDER D-800^{®} de la société TOSHIKI, et leurs mélanges.

Une charge peut être présente dans une composition cosmétique conforme à l'invention à raison de 0,5 à 40 % en poids de charge par rapport au poids total de la composition, de préférence de 5 à 30 %.

Une charge convenant à l'invention peut être par exemple une charge dont la granulométrie moyenne est inférieure à 100 µm, notamment comprise entre 1 et 50 µm, par exemple entre 4 et 20 µm.

### ADDITIFS

Les compositions cosmétiques selon l'invention peuvent également comprendre en outre tout additif usuellement utilisé dans le domaine concerné, choisi parmi des agents tensioactifs, des agents gélifiants, des agents filmogènes, et le cas échéant des auxiliaires de filmification, des gommes, des agent antioxydants, des huiles essentielles, des conservateurs, des parfums, des neutralisants, des agents hydratants, des agents antiseptiques, des vitamines telles que les vitamines B3 ou E et leurs dérivés, des agents protecteurs contre les UV, et leurs mélanges.

Les compositions selon l'invention peuvent être préparées de manière usuelle par l'homme du métier. Elles peuvent, ainsi, se présenter sous forme coulée et par exemple sous la forme d'un stick ou bâton, sous forme de pâte souple dans une bouillotte, ou sous la forme de coupelle utilisable par contact direct ou à l'éponge. Par exemple, elles peuvent constituer ensemble ou séparément, un fond de teint coulé, un fard à joues ou à paupières coulé, notamment coloré, un rouge à lèvres, un brillant pour les lèvres, un produit anti-cerne.

Chaque composition peut être conditionnée séparément dans un même article de conditionnement, par exemple dans un stylo bi-compartimenté, la composition de base pouvant être délivrée par une extrémité du stylo et la composition du dessus pouvant être délivrée par l'autre extrémité du stylo, chaque extrémité pouvant être fermée, par exemple de façon étanche, par un capuchon.

Par exemple, la composition qui est appliquée en première couche peut être sous forme solide ce qui peut permettre une application plus pratique, une meilleure stabilité dans le temps et en température de la composition, et peut permettre un tracé précis du maquillage, ce qui peut être hautement souhaitable dans le cas d'un rouge à lèvres ou d'un eye-liner.

Le produit selon l'invention peut être, par exemple, utilisé pour le maquillage et/ou le soin de la peau et/ou des lèvres selon la nature des ingrédients utilisés. Par exemple, le produit selon l'invention peut se présenter sous forme de fond de teint solide, de bâton ou de pâte de rouge à lèvres, de produit anticerne, ou de produit contour des yeux, d'eye-liner, de mascara, d'ombre à paupières, de produit de maquillage du corps ou un produit de coloration de la peau.

Par exemple, le produit selon l'invention peut être un produit de maquillage et/ou de soin des lèvres, par exemple un rouge à lèvres.

Selon un mode de réalisation, la première et/ou la seconde composition peu(ven)t être sous forme solide.

Selon un mode de réalisation, la couche inférieure appliquée sur la peau et/ou les lèvres peut présenter des propriétés de soin.

L'invention a encore pour objet un produit à lèvres, par exemple un rouge à lèvres comprenant une première et une seconde compositions telles que décrites précédemment.

Les compositions selon l'invention peuvent être obtenues par chauffage des différents constituants à la température de fusion des cires les plus élevées, puis coulage du mélange fondu dans un moule (coupelle ou doigt de gant). Elles peuvent également être obtenues par extrusion, comme décrit dans la demande EP A 0 667 146.

Selon un mode de réalisation, la présente invention a également pour objet un kit comprenant un produit conforme à la présente invention.

Un kit selon l'invention peut également comprendre des moyens d'application de la première et/ou de la seconde composition(s) sur la peau et/ou les lèvres.

A titre d'exemple de moyen d'application convenant à la mise en oeuvre de la présente invention, on peut mentionner des pinceaux, des brosses, des stylos, des crayons, des feutres, des plumes, des éponges, et des mousses.

Selon un mode de réalisation, un kit conforme à l'invention peut comprendre une première et une seconde composition conditionnées dans des compartiments ou récipients distincts.

Selon un mode de réalisation, la présente invention a encore pour objet un procédé de maquillage et/ou de soins de la peau et/ou des lèvres comprenant au moins une étape consistant à appliquer sur au moins une partie d'un support un produit conforme à l'invention.

Selon un mode de réalisation, un procédé conforme à l'invention peut être mis en oeuvre par application d'une composition comprenant un ester d'acide dimerdilinoléique et de polyols ou un de ses esters conforme à l'invention à titre de top-coat.

La présente invention a encore pour objet l'utilisation d'au moins un ester d'acide dimerdilinoléique et de polyols ou un de ses esters conforme(s) à la présente invention, en association avec un milieu physiologiquement acceptable pour préparer une première composition d'un produit cosmétique de soin et/ou de maquillage de la peau et/ou des lèvres comprenant en outre au moins une seconde composition comprenant un milieu physiologiquement acceptable, et présentant une tenue de la brillance améliorée.

Au sens de la présente invention, et sans indications contraires, « un » doit s'entendre comme signifiant « au moins un ».

Les exemples de compositions ci-après sont donnés à titre illustratif et sans caractère limitatif de l'invention.

### EXEMPLES

Les exemples de compositions selon l'invention, présentés ci-dessous, de type « base-coat » et « top-coat » peuvent être combinés l'un avec l'autre pour obtenir un produit cosmétique conforme à l'invention.

### Exemple 1 : Couche de base ("base-coat") solide

| Composé | % massique |
|---|---|
| Copolymère PVP/hexadécène | 1,00 |
| Tri-isostéarate de polyglycéryl-2 | 1,00 |
| Isononanoate d'isononyle | 1,00 |
| Dicaprate de néopentyle glycol | 1,00 |
| Phényl triméthicone | 1,00 |
| Polyisobutène hydrogéné | 4,00 |
| Isononanoate d'isononyle | 3,00 |
| Dicaprate de néopentyle glycol | 3,00 |
| Phényl triméthicone | 3,00 |
| Isododécane | 2,00 |
| D5 | 38,00 |
| PSPA | 4,00 |
| Résine MQ | 6,00 |
| BHT | 0,05 |
| Polyéthylène (M = 500) | 14,29 |
| Cire de candelilla | 1,90 |
| Dioxyde de titane | 0,19 |
| D&C Red 7 W 012 C | 0,43 |
| Oxydes de fer | 0,90 |
| Yellow 5 lake | 0,81 |
| Blue 1 lake | 0,19 |
| Copolymère di-méthacrylate d'éthylène glycol/méthacrylate de lauryle | 0,95 |
| N-lauroyl L-lysine | 0,95 |
| Hectorite quaternium-18 | 2,05 |
| Silice | 5,00 |
| Oxyde de fer recouvert de mica | 2,67 |
| Dioxyde de titane recouvert de borosilicate de calcium et d'aluminium | 0,48 |
| Dioxyde de titane recouvert de mica | 0,95 |
| Siméthicone | 0,19 |
| TOTAL | 100,00 |

### Mode opératoire

Une phase huileuse est préparée en mélangeant à chaud (environ 95 °C) les huiles.

La phase huileuse ainsi préparée est maintenue sous agitation à environ 95 °C et les charges sont ajoutées au mélange.

Puis sont rajoutés au mélange les cires, les pigments sous la forme d'une pâte pigmentaire, préparée par mélange des matières colorantes avec le triisostéarate de polyglycéryle et l'isononanoate d'isononyle, la siméthicone.

### Exemple 2 : Couche de base ("base-coat") liquide

| Composé | % massique |
|---|---|
| Copolymère PVP/hexadécène | 1,00 |
| Tri-isostéarate de polyglycéryl-2 | 1,00 |
| Isononanoate d'isononyle | 1,00 |
| Dicaprate de néopentyle glycol | 1,00 |
| Phényl triméthicone | 1,00 |
| Polyisobutène hydrogéné | 4,00 |
| Isononanoate d'isononyle | 3,00 |
| Dicaprate de néopentyle glycol | 3,00 |
| Phényl triméthicone | 3,00 |
| Isododécane | 2,00 |
| D5 | 38,00 |
| PSPA | 4,00 |
| Résine MQ | 6,00 |
| BHT | 0,05 |
| Cire micro-cristalline | 5,00 |
| Dioxyde de titane | 0,19 |
| D&C Red 7 W 012 C | 0,43 |
| Oxydes de fer | 0,90 |
| Yellow 5 lake | 0,81 |
| Blue 1 lake | 0,19 |
| Copolymère di-méthacrylate d'éthylène glycol/méthacrylate de lauryle | 0,95 |
| N-lauroyl L-lysine | 0,95 |
| Hectorite quaternium-18 | 11,24 |
| Silice | 7,00 |
| Oxyde de fer recouvert de mica | 2,67 |
| Dioxyde de titane recouvert de borosilicate de calcium et d'aluminium | 0,48 |
| Dioxyde de titane recouvert de mica | 0,95 |
| Siméthicone | 0,19 |
| TOTAL | 100,00 |

La composition a été préparée comme indiqué à l'exemple 1.

### Exemple 3 : Couche de dessus ("top-coat")

| Composé | % massique |
|---|---|
| Polyéthylène (PERFORMALENE 500 POLYETHYLENE-NEW PHASE TECHNOLOGIES) | 2,00 |
| Ozokérite | 3,00 |
| Cire d'abeille | 3,00 |
| Cire microcristalline | 2,00 |
| Cire petroleum (EMW-3 (NIPPON SEIRO)) | 8,00 |
| Polyisobutène hydrogéné | 10,00 |
| Malate de diisostéaryle | 10,00 |
| Dicaprate de néopentyl glycol | 10,00 |
| Isononanoate d'isotridécyl | 10,00 |
| Malate de diisostéaryl | 8,00 |
| Triisostéarate de polyglycéryl-2 (SALACOS 43N (NISSHIN OILLIO)) | 5,00 |
| Copolymère d'isostéarate de polyglycéryle-2 dimerdilinoléate (HAILUCENT ISDA) | 10,00 |
| Dimerdilinoleyldimerdilinoléate (LUSPLAN DD-DA7 (NFC)) | 2,00 |
| Bis-béhényl/Isostéaryl/Phytostéryl dimerdilinoléyle dimerdilinoléate (PLANDOL -G (NIPPON FINE CHEMICALS)) | 5,00 |
| Copolymère d'acrylate/acrylate de stéaryle/méthacrylate de diméthicone | 2,00 |
| Triméthylsiloxysilicate | 2,00 |
| Diméthicone | 0,90 |
| Mica recouvert de titane | 5,00 |
| Borosilicate de calcium d'aluminium (METASHINE : NIPPON SHEET GLASS) | 2,00 |
| D&C Red 28 | 0,10 |
| TOTAL | 100,00 |

Une phase huileuse est préparée en mélangeant à chaud (environ 95 °C) les huiles, ainsi que le copolymère d'isostéarate de polyglycéryl-2 dimerdilinoléate (HAILUSCENT ISDA).

La phase huileuse ainsi préparée est maintenue sous agitation à environ 95 °C et les charges sont ajoutées au mélange.

Puis sont rajoutés au mélange les cires, les pigments sous la forme d'une pâte pigmentaire, préparée par mélange des matières colorantes avec le triisostéarate de polyglycéryle-2 et l'isononanoate d'isononyle et la siméthicone, le bis-béhényl/isostéaryle/phytostéryle dimerdilinoléyle dimerdilinoléate (Plandool-G) et le Dimerdilinoleyldimerdilinoléate.

### Exemple 4 : Couche de dessus ("top-coat")

| Composé | % massique |
|---|---|
| Polyéthylène (PERFORMALENE 500 POLYETHYLENE - NEW PHASE TECHNOLOGIES) | 2,00 |
| Ozokérite | 3,00 |
| Cire d'abeille | 3,00 |
| Cire microcristalline | 2,00 |
| Cire petroleum (EMW-3 (NIPPON SEIRO)) | 6,00 |
| Polyisobutène hydrogéné | 10,00 |
| Malate de diisostéaryle | 10,00 |
| Dicaprate de néopentyl glycol | 10,00 |
| Isononanoate d'isotridécyl | 10,00 |
| Triisostéarate de polyglycéryl-2 (SALACOS 43N (NISSHIN OILLIO)) | 5,00 |
| Polyglycéryl-3-polydiméthyle siloxyéthyle diméthicone (KF6104 (SHINETSU)) | 5,00 |
| Diméthicone copolyol (KF6017 (SHINETSU)) | 5,00 |
| Copolymère d'isostéarate de polyglycéryle-2/dimerdilinoléate (HAILUCENT ISDA) | 10,00 |
| Dimerdilinoleyldimerdilinoléate (LUSPLAN DD-DA7 (NFC)) | 2,00 |
| Bis-béhényl/Isostéaryl/Phytostéryl dimerdilinoléyle dimerdilinoléate (PLANDOL -G (NIPPON FINE CHEMICALS)) | 5,00 |
| Copolymère d'acrylate/acrylate de stéaryle/méthacrylate de diméthicone | 2,00 |
| Triméthylsiloxysilicate | 2,00 |
| Diméthicone | 0,90 |
| Mica recouvert de titane | 5,00 |
| Borosilicate de calcium d'aluminium (METASHINE : NIPPON SHEET GLASS) | 2,00 |
| D&C Red 28 | 0,10 |
| TOTAL | 100,00 |

La composition a été préparée comme indiqué à l'exemple 3.

### Exemple 5 : Couche de dessus ("top-coat")

| Composé | % massique |
|---|---|
| Polyéthylène (PERFORMALENE 500 POLYETHYLENE - NEW PHASE TECHNOLOGIES) | 2,00 |
| Ozokérite | 3,00 |
| Cire d'abeille | 3,00 |
| Cire microcristalline | 2,00 |
| Cire petroleum (EMW-3 (NIPPON SEIRO)) | 6,00 |
| Polycottonseedate de sucrose | 2,00 |
| Polyisobutène hydrogéné | 10,00 |
| Malate de diisostéaryle | 10,00 |
| Dicaprate de néopentyl glycol | 10,00 |
| Isononanoate d'isotridécyl | 10,00 |
| Malate de diisostéaryle | 8,00 |
| Triisostéarate de polyglycéryl-2 (SALACOS 43N (NISSHIN OILLIO)) | 5,00 |
| Copolymère d'isostéarate de polyglycéryle-2/dimerdilinoléate (HAILUCENT ISDA) | 10,00 |
| Dimerdilinoleyldimerdilinoléate (LUSPLAN DD-DA7 (NFC)) | 2,00 |
| Bis-béhényl/Isostéaryl/Phytostéryl dimerdilinoléyle dimerdilinoléate (PLANDOL -G (NIPPON FINE CHEMICALS)) | 5,00 |
| Copolymère d'acrylate/acrylate de stéaryle/méthacrylate de diméthicone | 2,00 |
| Triméthylsiloxysilicate | 2,00 |
| Diméthicone | 0,90 |
| Mica recouvert de titane | 5,00 |
| Borosilicate de calcium d'aluminium (METASHINE : NIPPON SHEET GLASS) | 2,00 |
| D&C Red 28 | 0,10 |
| TOTAL | 100,00 |

La composition a été préparée comme à l'exemple 3.

## Revendications

1. Produit cosmétique de soin et/ou de maquillage de la peau et/ou des lèvres comprenant au moins :
- une première composition comprenant, dans un milieu physiologiquement acceptable, au moins un ester d'acide dimerdilinoléique et de polyol(s) ou un de ses esters, dont la viscosité, mesurée à environ 25 °C, est supérieure ou égale à environ 1 500 mPa.s, et
- une seconde composition comprenant un milieu physiologiquement acceptable.

2. Produit cosmétique de soin et/ou de maquillage de la peau et/ou des lèvres comprenant au moins deux compositions différentes et contenant respectivement au moins un ester d'acide dimerdilinoléique et de polyol(s) ou un de ses esters, dont la viscosité mesurée à environ 25 °C, est supérieure ou égale à environ 2 000 mPa.s.

3. Produit selon la revendication 2, dans lequel les esters contenus dans chacune des deux compositions sont différents.

4. Produit selon la revendication 1, 2 ou 3, dans lequel ledit ester possède un poids moléculaire variant d'environ 2000 à environ 25 000 g/mol, en particulier d'environ 5000 à environ 20 000 g/mol, en particulier d'environ 7000 à environ 15 000 g/mol, et plus particulièrement d'environ 8000 g/mol à environ 10 000 g/mol.

5. Produit selon l'une quelconque des revendications précédentes, dans lequel le polyol est un diol.

6. Produit selon la revendication précédente, dans lequel ledit ester comprend un enchaînement alterné de résidu(s) dimerdilinoléate(s) et de résidu(s) apparenté(s) au(x)dit(s) diol(s).

7. Produit selon la revendication 5 ou 6, dans lequel le diol est choisi parmi un dimère d'alcool gras, un mono- ou poly-glycérol, un mono- ou poly-alkylène en C₂₋₄ glycol, le 1,4 butanediol, et le pentaérythritol.

8. Produit selon la revendication 7, dans lequel le dimère d'alcool gras est le produit d'hydrogénation d'un dimère d'acide gras obtenu par dimérisation d'un acide gras insaturé en C₈ à C₃₄, notamment en C₁₂ à C₂₂, en particulier en C₁₆ à C₂₀ et plus particulièrement en C₁₈.

9. Produit selon l'une quelconque des revendications 6 à 8, dans laquelle chacune des deux extrémités dudit enchaînement porte respectivement un motif OR' et OR" avec R' et R" représentant, indépendamment l'un de l'autre, un atome d'hydrogène ou OR' et OR", représentant indépendamment l'un de l'autre, un résidu d'un monoalcool hydrocarboné en C₂ à C₃₆.

10. Produit selon la revendication 9, dans laquelle R' et R" représentent tous les deux un atome d'hydrogène.

11. Produit selon la revendication 9, dans laquelle OR' et OR" représentent tous les deux un résidu de monoalcool hydrocarboné, identique ou différent.

12. Produit cosmétique de soin et/ou de maquillage de la peau et/ou des lèvres comprenant au moins deux compositions différentes avec au moins une composition contenant au moins un ester de formule générale (I) suivante :
R₃-OCO-R₁(-COO-R₂-OCO-R₁)ₙ-COO-R₃ (I)
dans laquelle :
- OCR₁CO représente un résidu dimerdilinoléate,
- OR₂O représente un résidu de dimère d'alcool gras,
- OR₃ représente un résidu de monoalcool hydrocarboné, et
- n est un entier variant de 1 à 15.

13. Produit selon l'une quelconque des revendications 1 à 9 et 11, comprenant un ester tel que défini selon la revendication 12.

14. Produit selon la revendication 12 ou 13, dans laquelle OR₂O représente un résidu dimerdilinoléyle.

15. Produit selon l'une quelconque des revendications 12 à 14, dans laquelle OR₃ représente un résidu de monoalcool hydrocarboné choisi parmi les résidus béhényle, isostéaryle, phytostéryle et leurs mélanges.

16. Produit cosmétique de soin et/ou de maquillage de la peau et/ou des lèvres comprenant au moins deux compositions différentes avec au moins une composition contenant au moins un ester de formule générale (II) suivante : 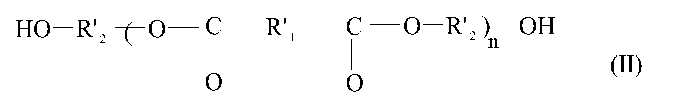 dans laquelle :
- n est un entier variant de 1 à 15,
- OCR'₁CO représente un résidu dimerdilinoléate,
- OR'₂O représente un résidu diglycéryle de formule générale (III) suivante : 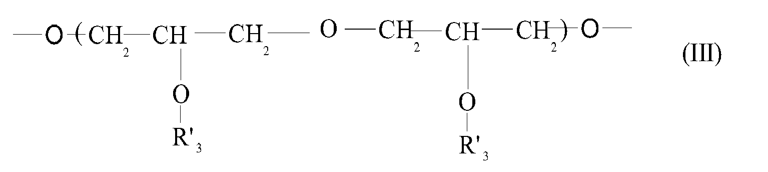
dans laquelle :
- R'₃ représente H ou OR'₃ représente un résidu d'acide gras.

17. Produit selon l'une quelconque des revendications 1 à 10, comprenant un ester tel que défini selon la revendication 16.

18. Produit selon la revendication 16 ou 17, dans laquelle le résidu d'acide gras figuré par OR'₃ est un résidu d'isostéaryle.

19. Produit cosmétique de soin et/ou de maquillage de la peau et/ou des lèvres comprenant au moins deux compositions différentes avec au moins une composition contenant au moins un ester de formule générale (IV) :
HO-R₁"-(-OCO-R₂"-COO-R₁"-)ₕ-OH (IV)
dans laquelle :
- OR₁"O représente un résidu de dimère diol obtenu par hydrogénation d'un acide dimerdilinoléique,
- COR₂"CO représente un résidu dimerdilinoléate hydrogéné, et
- h représente un entier variant de 1 à 9.

20. Produit selon l'une quelconque des revendications 1 à 10, comprenant un ester tel que défini en revendication 19.

21. Produit selon l'une quelconque des revendications précédentes, dans lequel ledit ester est choisi parmi les esters de nomenclature INCI suivante : le copolymère d'isostéarate de polyglycéryle-2/dimerdilinoléate, le bis-béhényl/isostéaryl/phytostéryl dimerdilinoléyle dimerdilinoléate, le dimerdilinoléyle dimerdilinoléate, et leurs mélanges.

22. Produit selon l'une quelconque des revendications précédentes, **caractérisé en ce que** la ou les compositions comprennent de 5 à 90 %, en particulier de 15 à 80 %, et plus particulièrement de 20 à 50 % en poids dudit ester par rapport au poids total de la composition.

23. Produit selon l'une quelconque des revendications précédentes, dans lequel lesdites compositions comprennent un milieu physiologiquement acceptable.

24. Produit selon la revendication 23, dans lequel le milieu physiologiquement acceptable comprend en outre, au moins une phase grasse liquide choisie parmi des huiles volatiles, des huiles non volatiles, et leurs mélanges.

25. Produit selon la revendication précédente, dans lequel l'huile volatile est choisie parmi :
- les huiles hydrocarbonées ayant de 8 à 16 atomes de carbone, et notamment les alcanes ramifiés en C₈-C₁₆ comme l'isododécane, l'isodécane, l'isohexadécane,
- les huiles de silicones linéaires ou cycliques, notamment celles ayant une viscosité ≤ 8 centistokes (8 10⁻⁶ m²/s), et en particulier l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane,
- les huiles fluorées volatiles telles que le nonafluorométhoxybutane ou le perfluorométhylcyclopentane, et
- leurs mélanges.

26. Produit selon la revendication 24, dans lequel l'huile non volatile est une huile de masse molaire allant d'environ 650 à environ 10 000 g/mol, en particulier d'environ 750 à environ 7500 g/mol, et plus particulièrement allant d'environ 1000 à environ 5000 g/mol.

27. Produit selon la revendication précédente, dans lequel ladite huile est choisie parmi :
- les polymères lipophiles,
- les esters d'acides gras linéaires ayant un nombre total de carbone allant de 35 à 70,
- les esters hydroxylés,
- les esters aromatiques,
- les esters d'alcools gras ou d'acides gras ramifiés en C₂₄-C₂₈,
- les huiles siliconées,
- les huiles d'origine végétale,
- et leurs mélanges.

28. Produit selon la revendication 26 ou 27, dans lequel ladite huile est choisie parmi les polybutylènes, les polyisobutylènes hydrogénés, les polydécènes, les polydécènes hydrogénés, les copolymères de la vinylpyrrolidone tel que le copolymère PVP/héxadécène, le tétrapélargonate de pentaérythrityle, le triisostéarate de polyglycérol-2, le tridécyl trimellitate, le citrate de triisoarachidyle, le tétraisononanoate de pentaérythrityle, le triisostéarate de glycéryle, le tri décyl-2 tétradécanoate de glycéryle, le tétraisostéarate de pentaérythrityle, le tétraisostéarate de polyglycéryle-2, le tétra décyl-2 tétradécanoate de pentaérythrityle, les silicones phénylées, l'huile de sésame, les huiles d'ester de dimer d'acide et d'alcool, et leurs mélanges.

29. Produit selon l'une quelconque des revendications 1 et 23 à 28, dans lequel le milieu physiologiquement acceptable comprend, en outre, au moins une phase grasse solide choisie parmi les corps gras pâteux, les cires, et leurs mélanges.

30. Produit selon l'une quelconque des revendications 1 et 23 à 29, dans lequel le milieu physiologiquement acceptable comprend en outre au moins une matière colorante.

31. Produit selon la revendication précédente, dans lequel la matière colorante est choisie parmi les matières colorantes organiques, les matières colorantes inorganiques, telles que les pigments et les nacres, les matériaux à effet optique spécifique, et leurs mélanges.

32. Produit selon l'une quelconque des revendications 1 et 23 à 31, dans lequel le milieu physiologiquement acceptable comprend en outre un polymère semi-cristallin.

33. Produit selon la revendication précédente, dans lequel ledit polymère semi-cristallin possède un point de fusion inférieur à environ 50 °C.

34. Produit selon la revendication 32 ou 33, dans lequel ledit polymère semi-cristallin est un polymère comportant a) un squelette polymérique hydrocarboné ou siliconé et b) au moins une chaîne organique latérale cristallisable et/ou une séquence organique cristallisable faisant partie du squelette polymérique dudit polymère, ledit polymère ayant une masse moléculaire moyenne en nombre supérieure ou égale à environ 2000.

35. Produit selon l'une quelconque des revendications 32 à 34, dans lequel ledit polymère semi-cristallin est choisi parmi les copolymères résultant de la polymérisation (a) d'un ou plusieurs monomère(s) carboxylique(s) avec (b) une ou plusieurs chaîne(s) polydiméthylsiloxane comportant au moins un radical polymérisable.

36. Produit selon la revendication précédente, dans lequel le monomère est choisi parmi l'acide acrylique, l'acide méthacrylique, l'acide maléique, l'acide fumarique, l'acide itaconique, l'acide crotonique, leurs esters, tels que les acrylates ou méthacrylates de méthyle, d'éthyle, de stéaryle, de butyle, d'éthyl-2-hexyle et leurs mélanges.

37. Produit selon l'une quelconque des revendications 32 à 36, dans lequel le polymère semi-cristallin est un copolymère d'acrylate et de diméthicone.

38. Produit selon l'une quelconque des revendications précédentes, dans lequel au moins une des deux compositions se présente sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de stick, de pâte, de mousse, de dispersion de vésicules notamment de lipides ioniques ou non, de lotion biphase ou multiphase, ou de poudre.

39. Produit selon l'une quelconque des revendications précédentes, dans lequel au moins une des deux compositions est anhydre.

40. Produit selon l'une quelconque des revendications précédentes, **caractérisé en ce qu'**il s'agit d'un produit de maquillage et/ou de soin des lèvres.

41. Kit de maquillage comprenant au moins un produit selon l'une quelconque des revendications 1 à 40.

42. Kit selon la revendication précédente, **caractérisé en ce qu'**il contient des moyens d'application de la première et/ou de la seconde composition(s) sur la peau et/ou les lèvres.

43. Kit selon la revendication 41 ou 42, **caractérisé en ce qu'**il contient des moyens d'application choisis parmi des pinceaux, des brosses, des stylos, des crayons, des feutres, des plumes, des éponges, et des mousses.

44. Kit selon l'une quelconque des revendications 41 à 43, **caractérisé en ce que** les première et seconde compositions sont conditionnées dans des compartiments ou récipients distincts.

45. Procédé de maquillage et/ou de soin de la peau et/ou des lèvres comprenant au moins une étape consistant à appliquer sur au moins une partie d'un support un produit tel que défini selon l'une quelconque des revendications 1 à 40.

46. Procédé selon la revendication précédente, dans lequel la composition contenant ledit ester est appliquée à titre de top-coat.
